# DEMANDE DE BREVET EUROPEEN

(11) **EP 4 722 335 A1**
(43) Date de publication de la demande: **08.04.2026**
(21) Numéro de dépôt: 25206719.4
(22) Date de dépôt: 03.10.2025
(51) Int. Cl.: C12M 1/22, C12M 3/00, C12M 1/00, C12M 1/36, C12M 1/34

(54) **MACHINE AUTOMATISÉE DE TRAITEMENT DE BOITES DE CULTURE MICROBIOLOGIQUE ET PROCÉDÉ DE TRAITEMENT CORRESPONDANT**

(30) Priorité: 03.10.2024 FR 2410664; 01.10.2025 FR 2511270
(71) Demandeur: Alliance Bio Expertise, 35170 Bruz (FR)
(72) Inventeur: FOURMONT, Arnaud, 35700 Rennes (FR); DEVELON, Johan, 35650 Le Rheu (FR); BONILLA, Stéphane, 35700 Rennes (FR)
(74) Mandataire: Vidon Brevets & Stratégie

(57) **Abrégé**

La présente invention concerne une machine automatisée de traitement de boîtes de culture, comprenant une unité opératoire destinée à recevoir les boîtes de culture en succession continue et à effectuer une série de traitements successifs sur chacune des boîtes de culture, ladite unité opératoire comprenant une pluralité de postes de traitement stationnaires (PT1-PT8) répartis selon un pas prédéterminé autour d'un axe (X2) et susceptibles d'être activés de façon au moins partiellement concomitante, et un dispositif d'entrainement en rotation (DER) des boîtes de culture par rapport aux postes de traitement, doté de logements répartis selon le pas des postes de traitement de sorte qu'une rotation du dispositif d'entrainement autour de l'axe amène une boîte de culture donnée successivement sur chacun des postes de traitement.

## Description

### Domaine technique

L'invention s'inscrit dans le domaine de l'instrumentation destiné aux laboratoires de biologie et de microbiologie. Plus particulièrement, l'invention porte sur une solution d'automatisation pour matériel de laboratoire adaptée pour la lecture et le tri de boîtes de culture biologique ou microbiologique.

L'invention trouve de nombreuses applications notamment, mais non exclusivement, dans les secteurs pharmaceutiques, cosmétiques, agro-alimentaires, vétérinaires et cliniques.

### Arrière-plan technologique

On s'attache plus particulièrement dans la suite de ce document à décrire la problématique existant dans le domaine de l'analyse microbiologique alimentaire. L'invention ne se limite bien sûr pas à ce contexte particulier d'application, mais présente un intérêt pour toute solution d'automatisation de laboratoire devant faire face à une problématique proche ou similaire.

La demande d'automatisation des pratiques de laboratoire a augmenté de manière significative au cours des dernières années, en particulier dans le secteur de l'agro-alimentaire où la sécurité, la productivité et la fiabilité des processus sont devenues primordiales.

Afin de garantir la sécurité du consommateur, les aliments et leur environnement sont en effet régulièrement soumis à des contrôles microbiologiques, à différents stades de la production. L'objectif de ces contrôles est d'obtenir le plus efficacement possible et en toute sécurité des informations relatives à la présence ou non de micro-organismes pathogènes dans les échantillons alimentaires prélevés et, le cas échéant, des informations relatives à la quantité de ces micro-organismes pathogènes, couramment appelés « colonies ».

Les laboratoires doivent traiter, chaque jour, une quantité substantielle d'échantillons, typiquement entre 1 000 et 10 000 boîtes de Petri par jour en fonction de leur taille et des besoins. Or, les différentes étapes de ce traitement sont encore réalisées la plupart du temps manuellement par un technicien de laboratoire qualifié. Pour la recherche d'un micro-organisme pathogène donné, tel que par exemple la listeria monocytogenes (bactérie responsable de la listériose chez l'être humain), le technicien procède tout d'abord à la lecture du code-barre apposé sur la boîte de Petri à l'aide d'un lecteur de code-barre. Puis, le technicien procède à une analyse microbiologique de l'échantillon consistant à rechercher et à identifier d'éventuels micro-organismes pathogènes. Il en saisit ensuite manuellement les résultats sur un ordinateur. Un comptage des colonies peut également être opéré visuellement par le technicien. Enfin, ce dernier effectue un tri manuel des boîtes de Petri identifiées comme étant positives (c'est-à-dire avec présence des micro-organismes) ou négatives (c'est-à-dire avec absence des micro-organismes).

Ces opérations sont répétitives et chronophages, pouvant conduire à des erreurs de manipulation ou à des contaminations croisées entre échantillons, voire des pertes d'échantillon, ce qui n'est pas optimal. De plus, un des besoins des laboratoires est de pouvoir libérer du temps de travail aux techniciens qualifiés afin de le réallouer à des tâches à plus forte valeur ajoutée et moins rébarbatives.

Des dispositifs de lecture de boites de Petri existent depuis une quinzaine d'années sur le marché. Ces dispositifs sont pour la majorité spécifiquement conçus pour un type de micro-organismes relativement limité, reposant sur des conditions fixes et homogènes. Une limite majeure de ces dispositifs se situe dans leur incapacité à effectuer la totalité des tâches nécessaires au processus de lecture des boîtes. De fait, la cadence de lecture est relativement faible (de l'ordre de 8 à 10 secondes typiquement) car le technicien doit y ajouter les étapes nécessaires à l'identification de l'échantillon (lecture manuelle du code à barres, et à la saisi des résultats de lecture).

Face à la diversité des milieux de culture, des échantillons et des formes de colonies, divers paramètres doivent être réglés pour adapter l'analyse et le rendu du résultat. Au quotidien, même avec des modèles de réglage préconfigurés, il est nécessaire d'ajuster certains paramètres pour permettre une lecture et/ou un comptage précis des colonies présentes dans les boîtes de Petri. Le technicien est donc amené à devoir ajuster les paramètres de manière empirique.

Il existe par ailleurs, dans l'état de la technique, des appareils de traitement automatique de boîtes de Petri combinant une intelligence artificielle pour l'identification et le comptage des colonies à l'usage d'un bras robotisé pour le chargement et le déchargement des boîtes de Petri, en coopération avec le lecteur de boîtes de Petri. De tels appareils permettent d'atteindre des cadences de traitement comprises entre 100 et 150 boîtes par heure, mais celles-ci restent tout de même en deçà des objectifs de cadence visés par les laboratoires d'analyse microbiologique, en particulier dans le domaine de l'agroalimentaire où les cadences de traitement visées sont plutôt comprises entre 700 et 900 boîtes par heure (sauf à multiplier le nombre d'appareils au sein des laboratoires, ce qui n'est pas envisageable d'un point de vue coût et encombrement). De plus, l'utilisation d'un bras robotisé est de mise en œuvre relativement complexe et coûteuse.

Enfin, de nouvelles générations d'incubateurs ont été lancées récemment sur le marché, lesquelles reposent sur l'intégration de capteurs d'images vidéo haute gamme ultra-réactifs, visant à renseigner en quasi-temps réel sur la présence de colonies microbiennes dans des boîtes de Petri. La capacité de traitement de tels incubateurs reste néanmoins limitée à 500 boites/jour, pour un coût de mise en œuvre élevé.

Il existe donc un vrai besoin de fournir une solution d'automatisation efficace, robuste et polyvalente pour lire et trier des boîtes de culture, et en particulier qui permet d'atteindre un niveau de productivité supérieur à l'existant, tout en garantissant des résultats précis et reproductibles.

### Exposé de l'invention

La présente invention permet de proposer une solution visant à remédier aux inconvénients de l'art antérieur.

Dans un mode de réalisation particulier de l'invention, il est proposé une machine automatisée de traitement de boîtes de culture, caractérisée en ce qu'elle comprend :
- un premier carrousel de stockage monté mobile en rotation autour d'un premier axe et destiné au stockage de boîtes de culture à traiter;
- un second carrousel de stockage monté fixe autour d'un second axe ;
- une unité opératoire destinée à recevoir les boîtes de culture en succession continue depuis le premier carrousel et à effectuer une série de traitements successifs sur chacune des boîtes de culture reçues, ladite unité opératoire comprenant :
   ∘ une pluralité de postes de traitement stationnaires répartis selon un pas prédéterminé autour du second axe, au moins une partie desdits postes de traitement étant susceptibles d'être activés de façon au moins partiellement concomitante, lesdits postes de traitement comprenant :
      ▪ un poste de réception destiné à recevoir une boîte de culture à traiter depuis le premier carrousel ;
      ▪ un poste d'analyse et d'attribution destiné à analyser le contenu de la boîte de culture reçue et à lui attribuer un type donné parmi au moins deux types de boîte prédéfinis en fonction des résultats d'analyse ;
      ▪ au moins un premier et un deuxième poste d'évacuation destinés à évacuer la boîte de culture vers le premier ou le second carrousel de stockage en fonction du type attribué à ladite boîte de culture ;
   ∘ un dispositif d'entrainement en rotation des boîtes de culture par rapport aux postes de traitement, ledit dispositif d'entrainement comprenant des logements de boîtes de culture répartis selon un pas correspondant au pas des postes de traitement de sorte qu'une rotation du dispositif d'entrainement autour du second axe amène une boîte de culture donnée successivement sur chacun des postes de traitement.

Ainsi, l'invention repose sur une solution d'automatisation complète et efficace de l'ensemble des tâches nécessaire à l'analyse et au tri de boîtes de culture. Cette approche consiste à équiper la machine de traitement d'une unité opératoire à postes de traitement multiples, destinée à recevoir les boîtes de culture en succession continue et à effectuer de manière automatisée un cycle de traitements successifs sur chacune des boîtes, tout ou partie des postes de traitement pouvant être activés de façon au moins partiellement concomitante. Chaque poste de traitement remplit un rôle spécifique dans le cycle de traitement au fur et à mesure que les boîtes de culture sont amenées sur chacun des postes, ce permet de paralléliser les traitements à opérer sur les boîtes, et in fine d'optimiser la cadence de traitement, tout en garantissant des résultats d'analyse précis et reproductibles. En effet, la durée des traitements opérés n'est pas toujours identique, certains traitements peuvent être réalisés en même temps, mais naturellement si un traitement a une durée plus courte, il se terminera avant un traitement de durée plus longue ou commencera après le démarrage du poste à traitement long. Autrement dit, deux postes à durée de traitement identique seront activés de manière totalement concomitante, alors que pour deux postes à durées de traitement différentes, le poste à traitement court sera activé de manière partiellement concomitante au poste à traitement long. Ainsi, selon une mise en œuvre particulière, la durée de traitement entre deux entrainements en rotation des boîtes de culture est définie en fonction du poste nécessitant la durée de traitement la plus longue.

Selon une mise en œuvre particulière, chaque poste d'évacuation est dédié à un type distinct de boîte de culture (par exemple un premier poste est dédié aux boites positives et un deuxième poste aux boites négatives). Selon une variante de mise en œuvre, au moins de postes d'évacuation sont dédiés à un même type de boîte de culture (par exemple deux premiers postes dédiés aux boites « positives » et un deuxième poste dédié aux boites « négatives »), ce qui permet d'augmenter la capacité de stockage pour un type de boîte de culture donné.

Selon une caractéristique particulière, ledit dispositif d'entrainement comprend une platine d'entrainement supérieure et une platine d'entrainement inférieure disposées en parallèle l'une de l'autre et solidaires l'une de l'autre, les platines d'entrainement supérieure et inférieure étant montées mobiles en rotation autour du second axe et comprenant un ensemble de logements supérieurs et inférieurs de boîtes de Petri respectivement, les logements supérieurs et inférieurs étant régulièrement répartis en périphérie des platines d'entrainement supérieure et inférieure autour du second axe, et disposés en regard deux à deux.

Ainsi, la machine prévoit une double platine d'entrainement pour convoyer les boîtes de culture d'un poste de traitement à l'autre, avec une possibilité de positionnement sur deux niveaux distincts, tout en conservant l'appariement du fond de la boîte avec son couvercle.

Selon une caractéristique particulière, ledit dispositif d'entrainement comprend en outre une sole de glissement supérieure) montée fixe par rapport au second axe et s'étendant partiellement en face inférieure de la platine d'entrainement supérieure, et une sole de glissement inférieure montée fixe par rapport au second axe et s'étendant en face inférieure de la platine d'entrainement inférieure.

Selon une première mise en œuvre du dispositif d'entrainement, les boîtes de culture à traiter étant munies chacune d'un couvercle solidarisé à un fond et disposées dans une position dite à l'endroit dans le premier carrousel :
- les logements supérieurs sont dotés chacun d'un trou traversant dimensionné pour le passage d'une boîte de culture et dont l'extrémité inférieure se termine par un épaulement annulaire dimensionné pour retenir le couvercle dans le logement supérieur et autoriser une désolidarisation du fond par rapport au couvercle par effet de gravité en l'absence de la sole de glissement supérieure, la sole de glissement supérieure s'étendant partiellement sous la platine d'entrainement supérieure pour maintenir le fond solidaire du couvercle sur au moins un des postes de traitement ;
- les logements inférieurs sont dotés chacun d'un trou traversant dimensionné pour le passage d'un fond de boîte de culture, la sole de glissement inférieure s'étendant sous la platine d'entrainement inférieure pour maintenir le fond dans le logement inférieur ;

Cette mise en œuvre particulière permet un traitement de boîtes de culture disposées à l'endroit dans le premier carrousel de stockage. La platine supérieure est ici dédiée à l'entrainement en rotation de boîtes de culture fermées ou de couvercles en fonction du poste de traitement concerné et la platine inférieure à l'entrainement en rotation de fonds.

Selon une caractéristique particulière, les postes de traitement stationnaires comprennent en outre un poste d'ouverture de boîte de culture comprenant :
- un clapet monté mobile entre au moins une position de maintien du fond solidaire du couvercle et une position de libération permettant une désolidarisation du fond par rapport au couvercle ;
- un manipulateur monté mobile en translation entre au moins une position de prélèvement du fond et une position de dépose du fond sur la platine inférieure ;
le clapet comprenant une lumière d'introduction du manipulateur, ladite position de prélèvement étant prise lorsque le clapet est dans la position de maintien du fond après passage du manipulateur à travers la lumière d'introduction du clapet.

Selon une mise en œuvre particulière, le clapet monté mobile en rotation autour d'un axe horizontal pour passer de la position de maintien à la position de libération, et vice versa.

Selon une variante de mise en œuvre, le clapet monté mobile en rotation autour d'un axe vertical d'un actionneur lequel est configuré pour exercer une pression sur un pion disposé dans une ouverture traversant le dispositif d'entrainement, ledit pion exerçant une pression sur le couvercle lorsque le clapet est en position de libération. Les inventeurs ont découvert que cette configuration facilite grandement l'ouverture des boîtes de Petri, le taux de réussite d'ouverture de boîtes de Petri s'en trouvant grandement accru.

Selon une caractéristique particulière, lesdits au moins un premier et un deuxième postes d'évacuation comprennent chacun un organe manipulateur monté mobile en translation entre au moins une position de prélèvement du fond depuis la platine d'entrainement inférieure, une position intermédiaire de solidarisation du fond avec le couvercle au niveau de la platine d'entrainement supérieure, et une position de dépose du fond et du couvercle solidarisés dans le premier ou le second carrousel de stockage.

Selon une caractéristique particulière, les postes de traitement stationnaires comprennent en outre au moins un poste d'identification de boîte de culture appartenant au groupe comprenant : un poste d'identification à lecteur de code-barre frontal et un poste d'identification à lecteur de code-barre latéral, ladite sole de glissement supérieure couvrant la face inférieure de la platine d'entrainement supérieure sur le poste de réception et ledit au moins un poste d'identification. Cela permet à la machine d'identifier chaque boîte de culture en cours de traitement et de l'associer avec le type qui lui est attribué.

Selon une caractéristique particulière, ledit au moins un premier poste d'évacuation est destiné à évacuer vers le premier carrousel les boîtes de culture pour lesquelles un premier type est attribué (par exemple les boîtes détectées négatives à la présence d'un microorganisme recherché) et ledit au moins un deuxième poste d'évacuation est destiné à évacuer vers le second carrousel les boîtes de culture pour lesquelles est attribué un deuxième type (par exemple les boîtes détectées positives à la présence d'un microorganisme recherché).

Ainsi, ledit dispositif d'entrainement est configuré pour coopérer d'une part avec le premier carrousel de stockage pour une boîte amenée sur ledit au moins un premier poste d'évacuation et d'autre part avec le deuxième carrousel de stockage pour une boîte amenée sur ledit au moins un deuxième poste d'évacuation. Le tri entre des boîtes de premier et de deuxième types peut ainsi être réalisé de manière totalement autonome par la machine en fonction des résultats d'analyse.

Selon une caractéristique particulièrement intéressante, le poste d'ouverture de boîte de culture est en outre destiné à évacuer vers le premier carrousel les boîtes de culture pour lesquelles un troisième type est attribué relatif à une problématique de traitement, le manipulateur (M4) dudit poste d'ouverture étant en outre monté mobile en translation entre une position, parmi ladite position de prélèvement et ladite position de dépose, et une position d'évacuation de boîte dans le second carrousel de stockage.

Ainsi, le poste d'ouverture de la machine remplit deux fonctions : une première fonction d'ouverture de boîte de culture pour les boîtes de culture ne posant pas de problématique de traitement et une deuxième fonction d'évacuation de boîte de culture dans le second carrousel pour les boîtes de culture problématiques. On entend par « problématique », une boîte de culture pour laquelle le code-barre n'a pas pu être lu par ledit au moins un poste d'identification disposé en amont dans le cycle de traitement ou une boîte de culture pour laquelle pour laquelle le couvercle n'a pas pu se désolidariser de son fond au poste d'ouverture. Une telle approche permet un tri plus poussé des boîtes de culture en mettant de côté celles posant un problème dans le cycle de traitement.

Selon une deuxième mise en œuvre du dispositif d'entrainement, les boîtes de culture à traiter étant munies chacune d'un couvercle solidarisé à un fond et disposées dans une position dite à l'envers dans le premier carrousel :
- les logements supérieurs sont dotés chacun d'un trou traversant dimensionné pour le passage d'une boîte de culture, la sole de glissement supérieure s'étendant partiellement sous la platine d'entrainement supérieure pour maintenir la boîte de culture fermée en position à l'envers dans le logement supérieur sur au moins un des postes de traitement ;
- les logements inférieurs sont dotés chacun d'un trou traversant dimensionné pour le passage d'une boîte de culture, la sole de glissement inférieure s'étendant sous la platine d'entrainement inférieure pour supporter le fond maintenir la boîte de culture fermée en position à l'envers dans le logement inférieur ;

Cette variante de mise en œuvre permet un traitement de boîtes de Petri disposées à l'envers dans le premier carrousel de stockage. Ainsi, l'invention offre la possibilité de réaliser un traitement sur des boîtes de culture pouvant être positionnées à l'endroit ou à l'envers, simplement en interchangeant la double platine d'entrainement. La machine selon l'invention est donc particulièrement polyvalente.

Dans un autre mode de réalisation de l'invention, il est proposé un procédé de traitement de boîtes de culture au moyen d'une machine de traitement automatisée comprenant un premier carrousel de stockage, un second carrousel de stockage, et une unité opératoire destinée à recevoir les boîtes de culture en succession continue et à effectuer une série de traitements successifs sur chacune des boîtes de culture, l'unité opératoire comprenant une pluralité de postes de traitement stationnaires répartis à un pas constant autour d'un axe de rotation et un dispositif d'entrainement en rotation des boîtes de culture par rapport aux postes de traitement, ledit dispositif d'entrainement comprenant des logements de boîtes de culture répartis selon un pas correspondant au pas des postes de traitement de sorte qu'une rotation du dispositif d'entrainement autour du second axe amène une boîte de culture donnée successivement sur chacun des postes de traitement pour effectuer les étapes suivantes, de façon au moins partiellement concomitante :
- réception d'une boîte de culture à traiter depuis le premier carrousel ;
- analyse du contenu de la boîte de culture reçue et attribution à ladite boîte un type donné parmi au moins deux types de boîte prédéfinis en fonction des résultats d'analyse ;
- évacuation de la boîte de culture dont le contenu a été analysé vers le premier ou le second carrousel de stockage en fonction du type attribué à ladite boîte de culture.

Dans un autre mode de réalisation de l'invention, il est proposé un produit programme d'ordinateur, comprenant des instructions de code de programme pour la mise en œuvre du procédé précité dans l'un quelconque de ses différents modes de réalisation, lorsque ledit programme est exécuté sur un ordinateur.

Dans un autre mode de réalisation de l'invention, il est proposé un médium de stockage lisible par ordinateur et non transitoire, stockant un produit programme d'ordinateur précité.

### Liste des figures

D'autres caractéristiques et avantages de l'invention apparaîtront à la lecture de la description suivante, donnée à titre d'exemple indicatif et non limitatif, et des dessins annexés, dans lesquels :
- la figure 1 est une représentation schématique d'une machine automatisée de traitement selon un mode de réalisation particulier de l'invention ;
- la figure 2 est une vue en perspective illustrant un dispositif d'entrainement de boîtes de Petri équipant la machine illustrée sur la figure 1 ;
- la figure 3 est une vue éclatée du dispositif d'entrainement illustré sur la figure 2 ;
- la figure 4 est une vue en coupe illustrant la structure d'un logement du dispositif d'entrainement ;
- les figures 5, 6 et 7 illustrent, sous forme schématique et de manière séquentielle, l'étape de réception d'une boîte de Petri au sein de l'unité opératoire selon l'invention ;
- les figures 8, 9, 10 et 11 illustrent, de manière séquentielle, l'étape d'ouverture d'une boîte de Petri selon l'invention ;
- la figure 12 schématise de manière simplifiée le principe de séparation entre le couvercle et le fond d'une boîte de Petri au cours de l'étape d'ouverture selon l'invention ;
- la figure 13 illustrent, sous forme schématique, l'étape d'analyse du contenu d'une boîte de Petri selon l'invention ;
- la figure 14 est une vue partielle de la machine mettant en évidence deux postes d'évacuation de boîte de type positive selon l'invention ;
- les figures 15, 16, 17 et 18 illustrent, sous forme schématique et de manière séquentielle, l'étape d'évacuation d'une boîte de Petri sur l'un des carrousels de stockage selon l'invention ;
- la figure 19 illustre, sous forme schématique, l'étape de lecture d'un code-barre frontal selon l'invention ;
- la figure 20 illustre, sous forme schématique, l'étape de lecture d'un code-barre latéral selon l'invention ;
- la figure 21 est une vue éclatée d'un dispositif d'entrainement selon une variante de réalisation de l'invention ;
- la figure 22 représente la structure simplifiée d'une unité de traitement mettant en œuvre le procédé selon un mode de réalisation particulier de l'invention ;
- la figure 23 est une vue partielle de la machine de traitement illustrant le principe de transfert d'une boîte de culture entre le carrousel de stockage et le dispositif d'entraînement ;
- les figures 24A et 24B sont deux vues partielles de la machine illustrant le poste d'ouverture de boîte de Petri selon une variante de l'invention ;
- les figures 25A, 25B, 25C, 25D et 25E illustrent, de manière séquentielle, l'étape d'ouverture d'une boîte de Petri selon la variante illustrée aux figures 24A et 24B.

### Description détaillée de l'invention

Sur toutes les figures du présent document, les éléments et étapes identiques sont désignés par une même référence numérique.

L'invention repose sur une solution d'automatisation complète de l'ensemble des tâches nécessaire à la lecture et au tri de boîtes de culture. Son principe général consiste à équiper la machine de traitement de boîtes de culture d'une unité opératoire à postes de traitement multiples, destinée à recevoir les boîtes de culture en succession continue et à effectuer de manière automatisée un cycle de traitements successifs sur chacune des boîtes de culture reçues. Chaque poste de traitement remplit un rôle spécifique dans le cycle de traitement au fur et à mesure que les boîtes de culture sont amenées sur chacun des postes, une partie des postes de traitement étant susceptibles d'être activés de façon au moins partiellement concomitante. Une telle automatisation permet entre autres, de réduire le temps de lecture et de tri de boites de culture, de diminuer le taux d'erreurs et de contaminations croisées et d'augmenter la cadence de traitement.

On s'attache plus particulièrement dans la suite du document à décrire l'invention dans le cadre d'un traitement de boîtes de Petri destiné à une utilisation en laboratoire de microbiologie alimentaire. L'invention ne se limite bien sûr pas à ce domaine particulier d'application, mais présente un intérêt pour tous types de contenant de culture nécessitant une analyse biologique ou microbiologique.

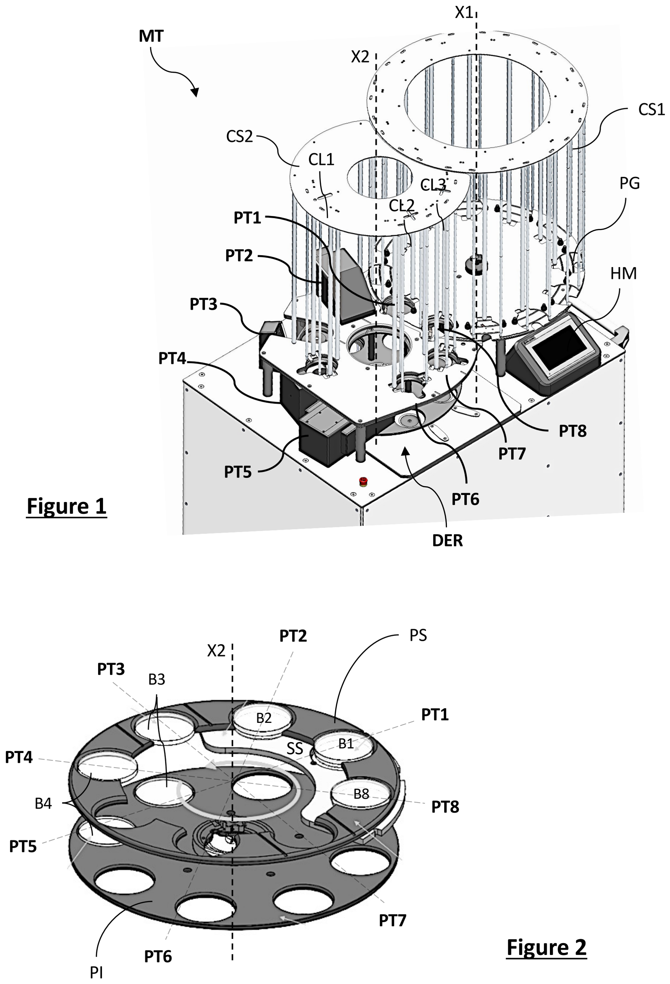

La **figure 1** présente une machine automatisée de traitement MT de boîtes de Petri selon un mode de réalisation particulier de l'invention. La machine de traitement MT est présentée à vide, sans boîtes de Petri, pour des questions de lisibilité de la figure. Il s'agit d'une machine programmable qui effectue de manière automatique des tâches répétitives, à grande vitesse et avec précision, pour trier les boîtes de Petri en fonction de leurs résultats d'analyse. Les boîtes présentant un résultat d'analyse positif à la présence de microorganisme(s) recherché(s) (listeria monocytogenes, Salmonelles, Escherichia coli ou Escherichia coli par exemple) sont dites boîtes « positives » et celles présentant un résultat d'analyse négative sont dites boîtes « négatives ».

La machine de traitement MT comprend plus particulièrement un premier carrousel de stockage CS1 monté mobile en rotation autour d'un premier axe X1, un second carrousel de stockage CS2 monté fixe autour d'un second axe X2, une unité opératoire disposée autour de l'axe X2 et une interface homme-machine HM. Dans cet exemple de réalisation, l'unité opératoire est composée de huit postes de traitement stationnaires PT1-PT8 et d'un dispositif d'entrainement en rotation DER permettant d'opérer un entrainement en rotation des boîtes de Petri autour de l'axe X2 par rapport aux postes de traitement PT1-PT8. L'ensemble de ces éléments est monté sur un châssis, muni de roulettes de déplacement à frein par exemple, pour faciliter le déplacement et la mise en place de la machine au sein du laboratoire.

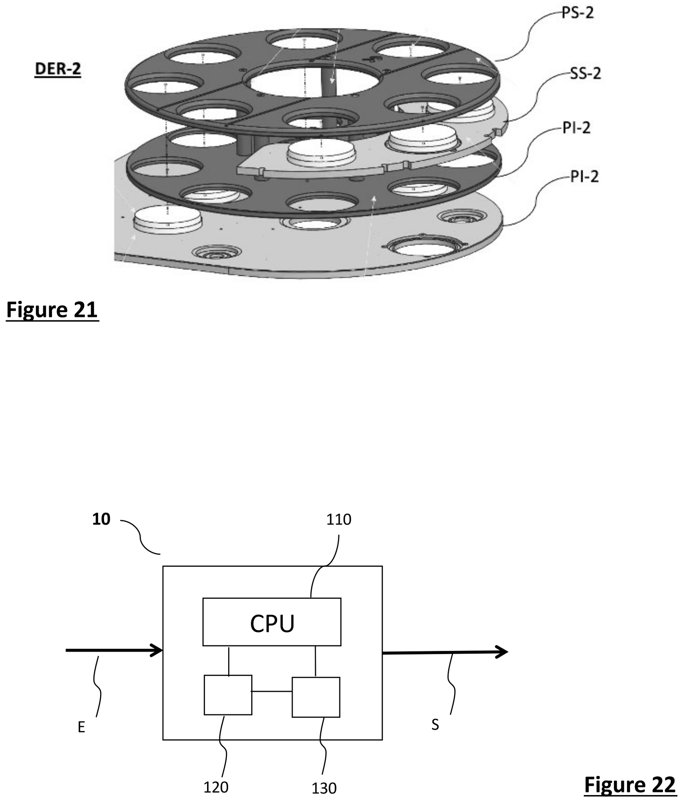

A l'intérieur du châssis sont installés les différents équipements de pilotage de ces éléments, en particulier les moteurs d'entraînement des pièces en mouvement et les composants électroniques constitutifs de l'unité matérielle pilotant la machine. Cette unité de pilotage (non représentée sur la figure) est raccordée électriquement au carrousel de stockage mobile CS1, aux différents éléments constituant le dispositif d'entrainement DER, ainsi qu'à l'interface HM, à des fins de pilotage de ces éléments. Le principe de fonctionnement de cette unité est détaillé plus loin en relation avec la **figure 22****.**

### Le carrousel de stockage CS1

Le carrousel de stockage CS1 est destiné au stockage des boîtes de Petri à traiter. Plus particulièrement, pour les besoins de l'invention, ce carrousel de stockage CS1 fait office à la fois de station distributrice des boîtes à traiter et de station réceptrice des boîtes négatives à la présence de listeria monocytogenes. Le carrousel CS1 présente une structure globalement cylindrique composée de deux plaques horizontales en regard l'une de l'autre et reliées entre elles par des trios de tiges verticales régulièrement répartis en périphérie de la structure autour de l'axe X1. Chaque trio de tiges, composé d'une tige rigide et de deux tiges flexibles par exemple disposées en triangle, est apte à recevoir une colonne de boîtes de Petri empilées. La partie supérieure des trios de tiges est faite dans une matière plus flexible que le reste pour faciliter la déformation élastique de celles-ci lors de l'insertion des boîtes de Petri par paquet. En variante, c'est la tige dans son ensemble qui est conçue dans un matériau souple adapté pour réaliser cette fonction. Le carrousel CS1 illustré ici possède douze colonnes de stockage angulairement réparties de manière régulière en périphérie de la structure. Chaque colonne peut contenir au moins quarante boîtes de Petri, de sorte que le carrousel CS1 peut contenir au moins quatre-cent quatre-vingts boîtes. La plaque inférieure du carrousel repose sur une plaque de glissement PG et est traversée, à chacune des colonnes de stockage, d'une ouverture autorisant le passage et le glissement d'une boîte de Petri sur la plaque de glissement PG. Le carrousel CS1 coopère avec un système d'entrainement en rotation (par exemple un moteur pas à pas relié mécaniquement à un moyeu solidaire de la plaque inférieure) pour rendre le carrousel mobile en rotation autour de l'axe X1 par rapport à la plaque de glissement PG selon un pas correspondant à 1/12^{ème} de tour, soit un pas d'angle de 2π/12 dans le sens anti-horaire. A noter que la plaque de glissement PG comprend également deux ouvertures, l'une autorisant le passage des boîtes à traiter du carrousel CS1 (depuis une colonne de stockage dite « distributrice ») selon l'axe vertical XD à l'unité opératoire, et l'autre autorisant le passage des boîtes négatives de l'unité opératoire au carrousel CS1 (dans une colonne de stockage dite « réceptrice ») selon l'axe vertical XR.

### Le carrousel de stockage CS2

Le carrousel de stockage CS2 est destiné au stockage des boîtes de Petri positives à la présence de listeria monocytogenes. Contrairement au carrousel de stockage CS1 qui est monté mobile en rotation, le carrousel CS2 est monté fixe autour de l'axe X2. Le carrousel CS2 est composé de deux plaques horizontales en regard l'une de l'autre et reliées entre elles par des jeux de tiges verticales régulièrement répartis en périphérie de la structure, formant les colonnes des boîtes de Petri positives de la machine. Les tiges verticales sont flexibles dans leur partie supérieure afin de pouvoir s'écarter et faciliter le retrait des boîtes de Petri par pile de boîtes empilées. Le carrousel CS2 représenté ici possède trois colonnes réceptrices CL1-CL3 réparties autour de l'axe X2 associées respectivement aux postes de traitement PT4, PT6 et PT7 de l'unité opératoire. La colonne réceptrice CL1 est prévue pour accueillir des boîtes « problématiques ». Les colonnes réceptrices CL2-CL3 sont quant à elles prévues pour accueillir les boîtes de Petri « positives ». La plaque inférieure du carrousel est traversée, à chacune des colonnes réceptrices, par une ouverture autorisant le passage des boîtes de Petri de l'unité opératoire au carrousel CS2.

### L'unité opératoire à postes de traitement multiples

L'unité opératoire est conçue pour recevoir les boîtes de Petri en succession continue depuis le carrousel de stockage CS1 et pour effectuer une série de traitements successifs sur chacune des boîtes de culture reçue, au moyen des postes de traitement stationnaires PT1-PT8 et du dispositif d'entrainement DER.

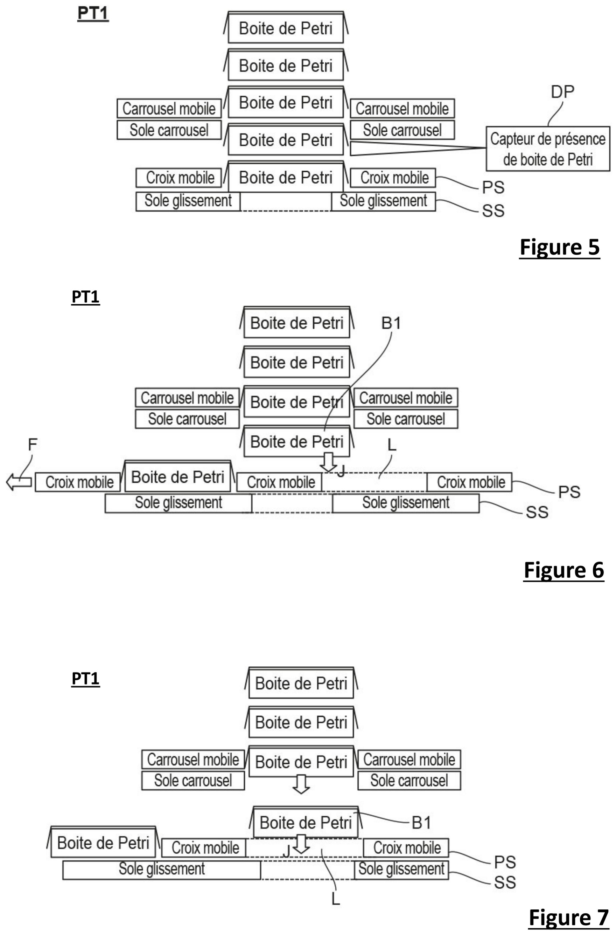

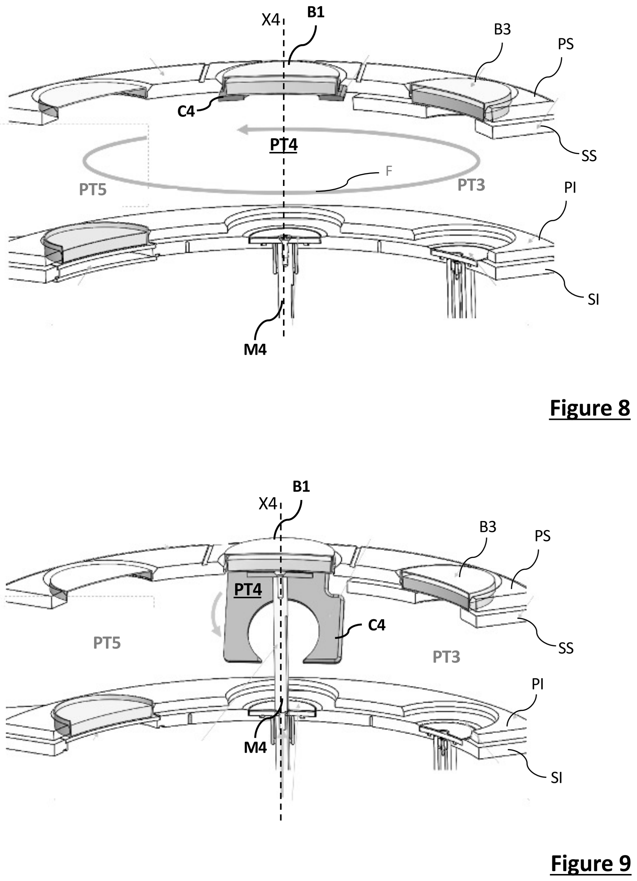

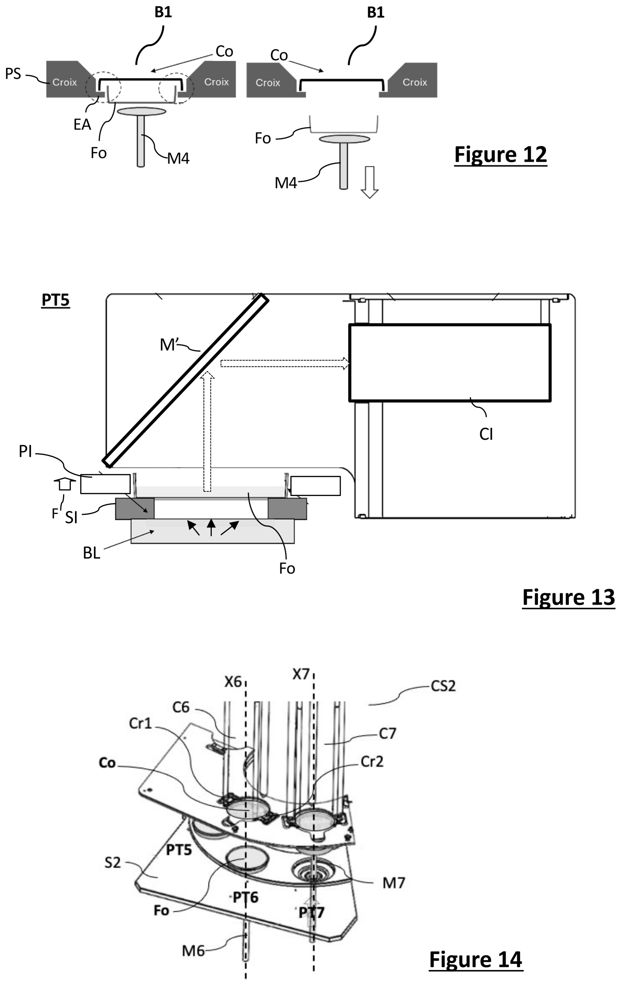

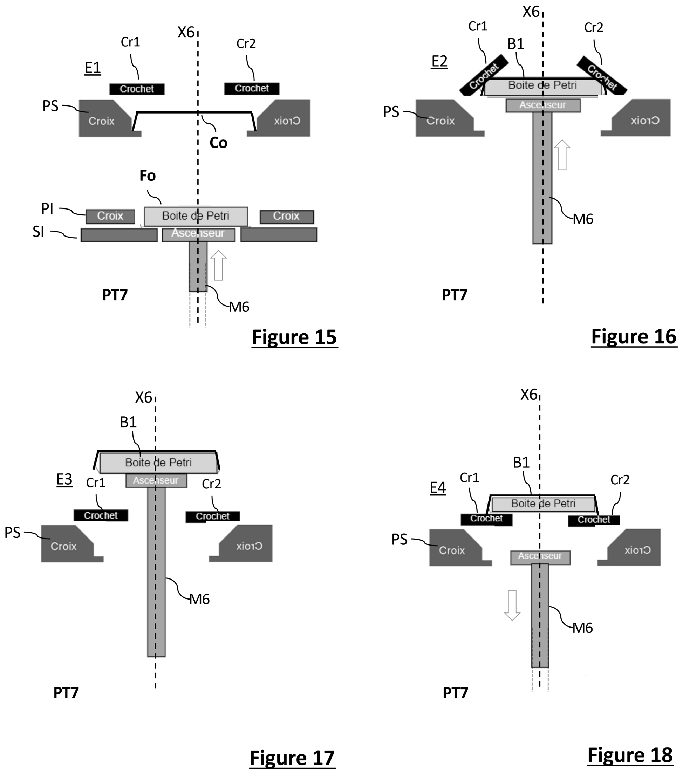

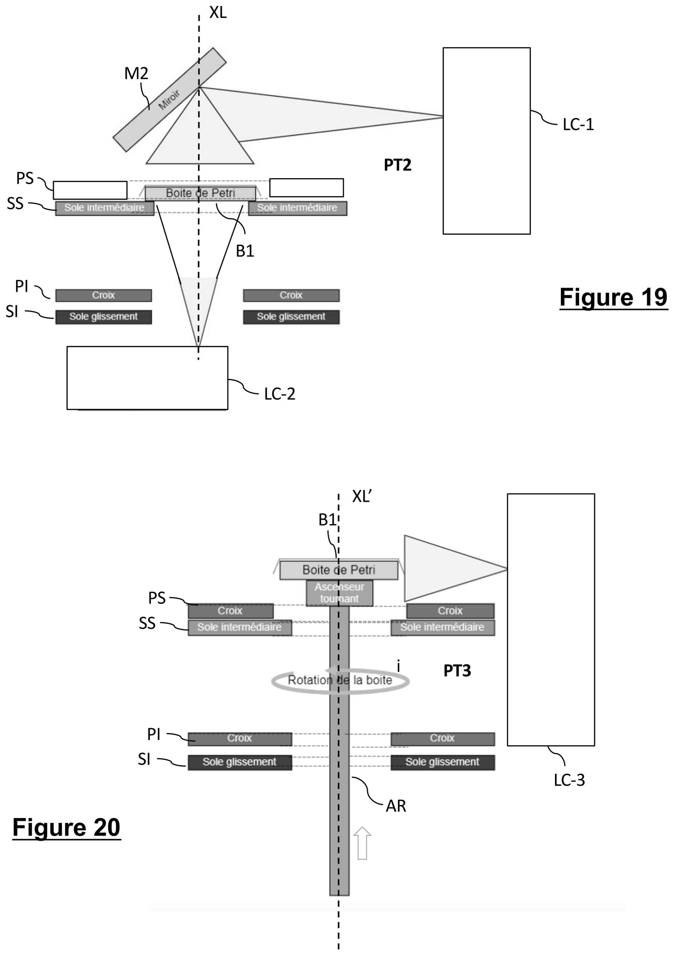

L'unité opératoire comprend, pour une boîte de Petri donné :
- un poste de réception **PT1** destiné à recevoir la boîte de Petri depuis le carrousel de stockage CS1 (principe de fonctionnement décrit plus loin en relation avec les **figures 5 à 7****)** ;
- un poste de lecture de code-barres frontal **PT2** destiné à lire un code-barres susceptible d'être apposé sur le couvercle ou sur le fond de la boîte de Petri (principe de fonctionnement décrit plus loin en relation avec la **figure 19****)** ;
- un poste de lecture de code-barres latéral **PT3** destiné à lire le code-barres susceptible d'être apposé latéralement sur la boîte de Petri (principe de fonctionnement décrit plus loin en relation avec la **figure 20****)** ;
- un poste d'ouverture de boîte de culture **PT4** destiné à désolidariser le couvercle du fond de la boîte de Petri (principe de fonctionnement décrit plus loin en relation avec les **figures 8 à 12****)** et à évacuer, le cas échéant, les boîtes de Petri « problématiques » ;
- un poste d'analyse et d'attribution **PT5** destiné à analyser le contenu de la boîte de Petri et à lui attribuer un type donné parmi au moins deux types de boîte prédéfinis (type positif ou type négatif) en fonction des résultats d'analyse (principe de fonctionnement décrit plus loin en relation avec la **figure 13****)** ;
- deux postes d'évacuation de boîtes positives **PT6-PT7** destinés chacun à évacuer la boîte de Petri vers le carrousel de stockage CS2 en cas de résultat positif à la présence de listeria monocytogenes attribué au poste PT5 (principe de fonctionnement décrit plus loin en relation avec la **figure 14****) ;**
- un poste d'évacuation de boîtes négatives **PT8** destinés à évacuer la boîte de Petri vers le carrousel de stockage CS1 en cas de résultat négatif à la présence de listeria monocytogenes attribué au poste PT5 (dont le principe de fonctionnement est écrit plus loin en relation avec les **figures 15 à 18****).**

Les postes de traitement PT1-PT8 sont dits « stationnaires » car ils sont montés fixes sur le châssis de la machine MT par rapport à l'axe X2. Les postes PT1-PT8 sont régulièrement répartis autour de l'axe X2 selon un pas sensiblement égal à 1/8^{ème} de tour, soit un pas d'angle de 2π/8.

Afin de gagner en cadence de traitement, les postes de traitement PT1-PT8 et l'unité de pilotage sont configurés, dans cet exemple de réalisation, pour que les postes de traitement soient susceptibles d'être activés de façon au moins partiellement concomitante. En effet, les postes de traitement à durées de traitement sensiblement identiques sont activés de façon concomitante sur l'ensemble de leur durée, alors que les postes de traitement à durées distinctes sont activés de façon partiellement concomitante (typiquement un poste à traitement court commencera après le démarrage d'un poste à traitement long ou se terminera avant la fin de celui-ci).

L'unité opératoire comprend en outre un dispositif d'entrainement DER de boîtes de Petri en rotation autour de l'axe X2. Un tel dispositif comprend huit logements de boîtes de Petri répartis selon un pas correspondant au pas des postes de traitement PT1-PT8, soit un pas d'un angle 2π/8, de sorte qu'une rotation du dispositif d'entrainement DER autour de l'axe X2 amène la boîte de Petri successivement sur chacun des postes de traitement PT1-PT8. Pour se faire, le dispositif d'entrainement DER coopère avec un système d'entrainement en rotation (détaillé ci-après) permettant d'animer les boîtes de Petri (disposés dans les logements) d'un mouvement de rotation discontinu, c'est-à-dire tournant pas à pas, par pas d'un angle de 2π/8 par rapport à la plaque inférieure du carrousel CS2.

On notera ainsi que la machine de traitement MT compte autant de boîtes de Petri en cours de traitement que de postes de travail compris dans l'unité opératoire. Tous les postes de travail fonctionnent en permanence sauf lorsque le dispositif d'entrainement DER est en cours de rotation pour acheminer les boîtes d'un poste à l'autre.

On décrit maintenant plus en détail, en relation avec les **figures 2** à **4****,** la structure du dispositif d'entrainement en rotation selon l'invention.

En préliminaires, il est important de noter que les boîtes de Petri sont des petites boîtes en matière plastique optiquement transparente, composées d'un fond et d'un couvercle. Chacune de ces parties possède une paroi latérale de différent diamètre, la paroi du couvercle venant recouvrir celle du fond lorsque la boîte est fermée. Cette mise en place se fait relativement sans frottement, si bien que le retrait du couvercle du fond de la boîte se fait sans effort (on parlera aussi de « désolidarisation » du fond par rapport au couvercle).

Dans cet exemple de réalisation, le dispositif d'entrainement DER comprend une double platine d'entrainement en rotation composée d'une platine d'entrainement supérieure PS et d'une platine d'entrainement inférieure PI. Les platines d'entrainement PS et PI sont disposées en parallèle l'une de l'autre et sont solidaires l'une de l'autre par des éléments de liaison, tels que des entretoises BL. Les platines d'entrainement PS et PI se présentent sous une forme globale de disque annulaire. Elles sont montées mobiles en rotation autour de l'axe X2 à l'aide d'un système d'entrainement en rotation, par exemple un moteur pas à pas relié mécaniquement à un moyeu central solidaire de la platine PI et/ou platine PS, aligné sur l'axe X2. Le moteur pas à pas est configuré pour animer la double platine d'un mouvement de rotation pas à pas, par pas d'angle de 2π/8, par rapport aux soles de glissement supérieure SS et inférieure SI du dispositif. La sole de glissement supérieure SS est montée fixe sur le châssis par rapport à l'axe X2 et s'étend partiellement sur la face inférieure de la platine supérieure SS de manière à maintenir le fond solidaire du couvercle des boîtes de Petri sur les postes PT1, PT2 et PT3. La sole de glissement inférieure SI est montée fixe sur le châssis par rapport à l'axe X2 et s'étend sur la face inférieure de la platine inférieure PI. Les soles de glissement SS et SI peuvent également être dotées de trous traversants en fonction du poste de traitement concerné, dimensionnés pour le passage d'un manipulateur (cas des postes PT4, PT6, PT7 et PT8) ou d'un faisceau de lumière de rétroéclairage (cas du poste PT5), tout en assurant le maintien du fond de boîte.

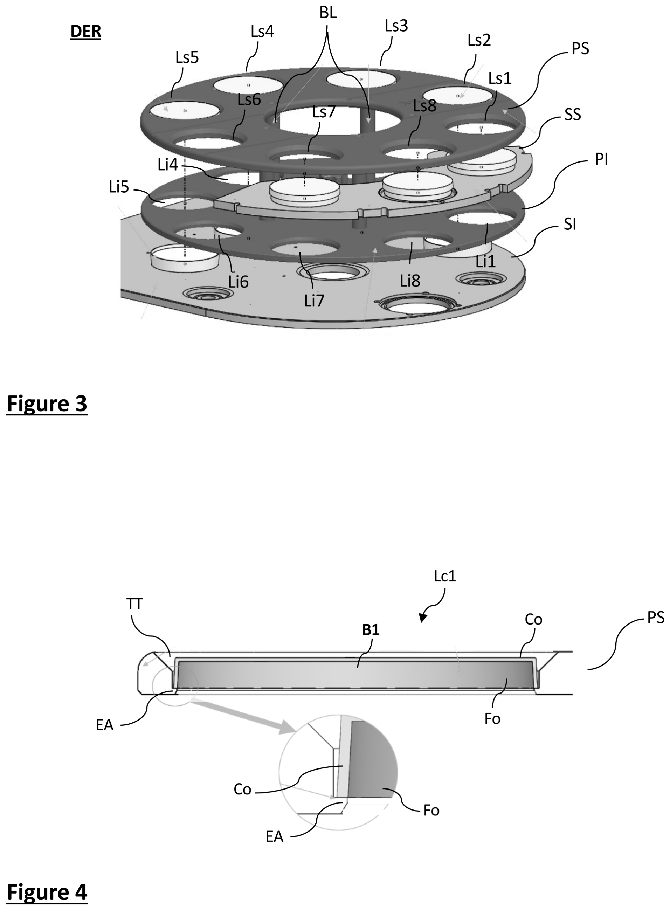

La platine supérieure PS comprend une série de huit logements supérieurs, référencés Ls1-Ls8 sur la **figure 3****,** régulièrement répartis selon un pas d'angle de 2π/8 en périphérie de platine supérieure PS autour de l'axe X2. La platine inférieure PI comprend une série de huit logements inférieurs, référencés Li1-Li8, régulièrement répartis selon un pas d'angle de 2π/8 en périphérie de platine autour de l'axe X2 dont les axes verticaux coïncident avec ceux des logements supérieurs Ls1-Ls8. Ainsi, grâce à la présence des entretoises, les logements Ls1-Ls8 de la platine supérieure et les logements Li1-Li8 de la platine inférieure sont donc disposés en regard deux à deux de façon à former des couples de logements supérieur-inférieur restant appairés tout au long du cycle de traitement.

Les logements supérieurs Ls1-Ls8 sont dotés chacun d'un trou traversant dimensionné pour autoriser le passage d'une boîte de Petri et se terminant par un épaulement annulaire EA, lequel est conformé pour retenir le couvercle de la boîte dans le logement supérieur et pour laisser passer le fond de la boîte par effet de gravité (en l'absence de sole de glissement). La **figure 4** illustre par exemple le détail du logement supérieur Ls1 accueillant une boîte de Petri B1. Le fond de la boîte est référencé Fo et le couvercle de la boîte, Co. Dans ce mode de réalisation particulier, la boîte de Petri B1 est disposée à l'endroit dans le logement Ls1. Ce dernier comprend un trou traversant TT comportant une première portion de forme conique (conduisant à une réduction de section progressive), une deuxième portion cylindrique, et se terminant par un épaulement annulaire EA de retenue de couvercle.

Quant aux logements inférieurs Li1-Li8, ils présentent chacun un trou traversant dimensionné pour permettre le passage du fond des boîtes de Petri au travers la platine d'entrainement inférieure PI, le fond des boîtes étant maintenus sur la sole de glissement SI. Ces trous permettent également le passage d'un manipulateur (cas des postes PT3, PT4, PT6, PT7 et PT8) ou le passage d'un faisceau de lumière arrière (cas du poste PT5).

Dans ce mode de réalisation particulier, la platine d'entrainement PS a donc pour fonction, en présence de la sole de glissement SS, d'amener une boîte de Petri complète (ensemble fond-couvercle) sur chacun des postes de traitement PT1, PT2 et PT8 (cas des boîtes B1, B2 et B8 sur la **figure 2****)** et, en présence de la sole de glissement SS, d'amener seul le couvercle de boîte sur chacun des postes de traitement PT3, PT4, PT5, PT6 et PT7 (cas des boîtes B3 et B4). Tandis que la platine d'entrainement PI a pour fonction de maintenir le fond des boîtes de Petri et d'amener celui-ci sur chacun des postes de traitement PT3, PT4, PT5, PT6 et PT7 (cas par exemple des boîtes de Petri B3 et B4). A noter que le fait de véhiculer des boîtes de Petri fermée lorsque le traitement le permet, réduit le risque de contamination croisée.

L'alignement deux à deux des logements supérieurs Ls1-Ls8 et inférieurs Li1-Li8 permet de conserver d'un poste de traitement à l'autre, et tout au long du cycle de traitement d'une boîte de Petri, l'appariment du couvercle avec son fond de boîte, même en cas d'arrêt de la machine MT. Ceci permet de diminuer le risque d'intervertir le couvercle et/ou le fond entre différentes boîtes de Petri, assurant ainsi la traçabilité de l'échantillon analysé (suivi porté par le code-barres apposé sur le fond et/ou le couvercle de la boîte).

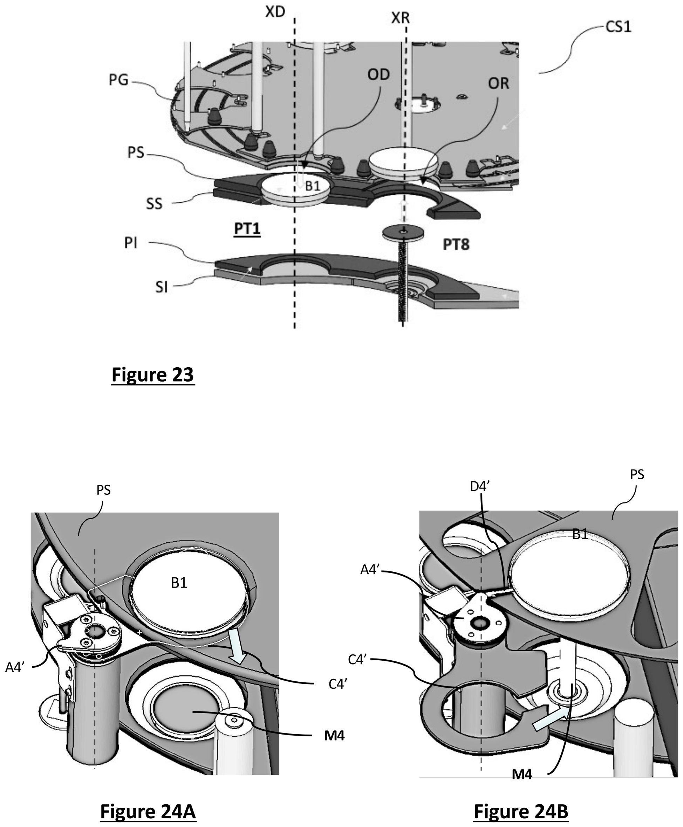

On notera que le carrousel CS1 et la double platine d'entrainement se chevauchent partiellement sur une partie de leur périphérie respective de sorte à permettre un alignement de l'ouverture de distribution OD - au niveau du poste de réception PT1 - avec l'un des couples de logements de la platine d'entrainement et un alignement de l'ouverture de réception OR - au niveau du poste d'évacuation PT8 - avec un autre de couple de logements de la platine d'entrainement (comme illustré plus en détail sur la **figure 23****).** En d'autres termes, le carrousel CS1 et la double platine d'entrainement PS-PI sont partiellement imbriqués de manière à faire coïncider les positions de leurs ouvertures lors de leur rotation respective.

### L'interface homme-machine HM

L'interface HM est le tableau de bord qui permet à l'utilisateur de communiquer avec la machine MT lui donnant des consignes en fonction du cycle de traitement qu'il souhaite réaliser et de répondre aux demandes d'instructions au microprocesseur. Ces consignes visent notamment : le nombre de boîtes à traiter, le type de gélose présente dans les boîtes de Petri, les paramètres d'imagerie et la nature des microorganismes recherchés. L'interface HM permet l'utilisation de l'invention en mode automatique ou semi-manuel. L'accès au mode automatique permet de lancer un traitement des boîtes de Petri une fois que celles-ci sont chargées dans le carrousel CS1. Le mode semi-manuel permet d'avoir accès à l'ensemble des postes de traitement et de les faire fonctionner indépendamment les uns des autres.

### Exemple de fonctionnement de la machine

On présente ci-après, en relation avec les **figures 5** à **20****,** le fonctionnement de la machine MT, étape par étape, sur un cycle de traitement complet et pour une boîte de Petri donnée. Ces étapes sont mises en œuvre par une unité de traitement de la machine (dont le principe est détaillé plus loin en relation avec la **figure 22****).**

Après avoir mis en place les boîtes de Petri dans le carrousel de stockage CS1, l'opérateur initialise un cycle de traitement via l'interface HM. On prendra garde de conserver au moins une colonne de stockage vide à disposition, dédiée à la réception et l'empilement des boîtes de Petri positives provenant de l'unité opératoire. De manière optionnelle, les boîtes de Petri peuvent être insérées dans les colonnes de stockage, non pas après, mais avant la mise en place effective des carrousels CS1 et CS2 sur le bâti de la machine.

On considère que les boîtes de Petri sont remplies d'un échantillon alimentaire pour lequel la présence de listeria monocytogenes est recherchée.

Au début de l'opération, en vue de rattraper les jeux éventuels entre les pièces, le carrousel CS1 et le dispositif d'entrainement sont mis brièvement en rotation de manière à réaliser un parfait centrage de la plaque de glissement PG et du carrousel CS1 dans l'axe X1, et un parfait centrage de la double platine PS-PI dans l'axe X2. Des capteurs sont prévus pour contrôler le bon positionnement des carrousels et du dispositif d'entrainement au démarrage et tout au long du cycle de traitement.

### Étape 1 : Transfert de la boîte de Petri vers l'unité opératoire (Figures 5-7, 23)

La première étape consiste à acheminer la boîte de Petri depuis le carrousel CS1 dans lequel elle est stockée vers le dispositif d'entraînement DER, au poste de travail PT1. Pour se faire, le carrousel CS1 et le dispositif d'entraînement DER sont chacun mis en rotation jusqu'à ce que l'axe vertical associé à la colonne de stockage ainsi que celui associé à la paire de logements de la double platine soient confondus avec l'axe d'ouverture de distribution XD. Une fois les ouvertures placées en vis-à-vis les uns des autres le long de l'axe XD, la boîte de Petri B1 situé en dessous de la colonne de stockage est automatiquement transférée, sous l'effet des forces de pesanteur agissant sur elle, dans le logement L de la platine supérieure PS au travers de l'ouverture de distribution OD. Le fond de la boîte repose alors sur la sole de glissement SS.

La présence de chanfreins sur les bords extérieurs des ouvertures du carrousel CS1 et la platine d'entrainement PS facilite le transfert de la boîte (passage progressif) en limitant les risques d'accrochage en particulier dans l'espace situé entre le carrousel de stockage et la platine d'entrainement.

La présence de la sole de glissement SS sert de support à la boîte de Petri (ou au fond de la boîte à tout le moins) dans le logement supérieur de la platine PS1 et permet son glissement, lorsque la platine supérieur PS est mise en rotation autour de l'axe X2 par rapport à la sole de glissement SS.

Un détecteur de présence DP, connecté à l'unité de traitement, est agencé pour détecter la présence d'une boîte de Petri dans l'ouverture OD en attente d'être transférée dans le logement suivant de la platine supérieure. La présence d'une boîte détectée par le détecteur DP déclenche le mouvement de la double platine en rotation d'un pas angulaire de 2π/8 autour de X2 dans le sens anti-horaire (représenté par la flèche F à la **figure 6****),** entrainant le déplacement de la boîte de Petri B1 du poste PT1 au poste PT2, ainsi que les autres boîtes déjà présentes dans le dispositif d'entrainement DER vers les postes de travail suivants. Une fois la rotation terminée, la boîte de Petri suivante de la colonne de stockage est automatiquement transférée dans le logement suivant de platine d'entrainement, et ainsi de suite. Les différentes boîtes de Petri stockées arrivent donc successivement au poste de PT1 (mouvement figuré par la flèche J aux figures 6 et 7).

Ainsi le mouvement de la double platine d'entrainement synchronisé avec le transfert d'une nouvelle boîte de Petri vers l'unité opératoire permet une automatisation du déplacement des boîtes de Petri d'un poste de travail à l'autre, depuis leur zone initiale de stockage jusqu'à leur zone finale de stockage.

A titre alternatif ou de manière complémentaire, un organe manipulateur mobile en translation selon l'axe XD peut être prévu pour faciliter le transfert des boîtes de Petri du carrousel CS1 au dispositif d'entrainement DER.

Lorsque la dernière boîte d'une colonne de stockage du carrousel CS1 est transférée dans l'unité opératoire, l'absence de présence de boite détectée par le détecteur DP entraîne le déplacement du carrousel CS1 d'un pas angulaire de 2π/12 autour de l'axe X1, afin de positionner une nouvelle pile de boîtes au-dessus de l'ouverture de distribution OD, et de continuer ainsi le transfert en succession continue des boîtes de culture encore stockées dans le carrousel CS1, vers l'unité opératoire. Si la nouvelle colonne de stockage qui se présente au-dessus de l'ouverture de distribution OD est vide, le système d'entrainement du carrousel CS1 est à nouveau activé d'un pas angulaire de 2π/12 autour de l'axe X1 pour traiter une nouvelle pile de boîtes, et ainsi de suite jusqu'à ce que toutes les colonnes distributrices soit passées.

La double platine est ensuite actionnée par l'unité de pilotage pour entrainer la boîte de Petri d'un pas angulaire de 2π/8 autour de l'axe X2 afin d'être acheminée du poste PT1 au poste PT2.

### Étape 2 : Lecture de code-barres frontal (Figure 19)

La deuxième étape consiste à réaliser une lecture du code-barres situé sur le couvercle ou le fond de la boîte de Petri. Cette deuxième étape est mise en œuvre lorsque la boîte de Petri est positionnée au poste PT2.

Le poste PT2 comprend un premier lecteur de code-barres LC-1, disposé en périphérie du dispositif d'entrainement DER et perpendiculairement à l'axe du logement XL, coopérant avec un miroir réfléchissant M, disposé au-dessus de la platine PS et dans l'axe XL, pour permettre une lecture optique frontal (plein champ ou par balayage) du code-barre porté par le couvercle de la boîte de Petri. A titre alternatif ou complémentaire, le poste PT2 est également équipé d'un second lecteur de code-barres LC-2 disposé en dessous du dispositif d'entrainement DER et agencé pour permettre une lecture optique arrière (plein champ ou par balayage) du code-barre porté par le fond de la boîte de Petri.

Les lecteurs de code-barres LC-1 et LC-2 de la machine sont activés lorsque la double platine a terminé sa rotation de 2π/8. La fonction de ce poste de travail est de permettre une lecture de code-barres et une identification de l'échantillon contenu dans la boîte de Petri quelle que soit la position du code-barres sur la boîte. Les données issues de la lecture du code-barres sont sauvegardées dans une mémoire de stockage de la machine. Elles permettent de suivre la boite de Petri tout au long du traitement sur les différents postes de travail de l'unité opératoire.

La double platine est ensuite actionnée par l'unité de pilotage pour entrainer la boîte de Petri d'un pas angulaire de 2π/8 autour de l'axe X2 afin d'être acheminée au poste PT3.

### Étape 3 : Lecture de code-barres latéral (Figure 20)

La troisième étape consiste à réaliser une lecture du code-barres situé sur la surface latérale de la boîte de Petri. Cette troisième étape est mise en œuvre lorsque la boîte de Petri est positionnée au poste PT3.

Le poste PT3 comprend un lecteur de code-barres latéral LC-3, disposé en périphérie du dispositif d'entrainement DER et perpendiculairement à l'axe du logement XL', un manipulateur M1 monté mobile en translation le long de l'axe XL' et coopérant avec le lecteur LC-3 pour permettre une lecture optique du code-barre porté par la tranche de la boîte de Petri. Le manipulateur M3 monté mobile en translation entre une position basse de maintien sur la platine PS et une position haute de lecture par le lecteur LC-3. Le manipulateur M3 est en outre monté mobile en rotation autour d'un axe pivotant à 360 degrés (mouvement matérialisé par la flèche i) ce qui permet au lecteur LC-3 de scanner la totalité de la surface de la tranche de la boîte de Petri, lorsque le manipulateur M3 est en position de lecture, afin de s'assurer la lecture du code-barres.

La double platine est ensuite actionnée par l'unité de pilotage pour entrainer la boîte de Petri d'un pas angulaire de 2π/8 autour de l'axe X2 afin d'être acheminée au poste PT4.

### Étape 4 : Ouverture de la boîte de Petri ou évacuation (Figures 8 à 12)

La quatrième étape consiste à désolidariser le couvercle du fond de la boîte de Petri. Cette quatrième étape est réalisée lorsque la boîte de Petri est positionnée au poste PT4.

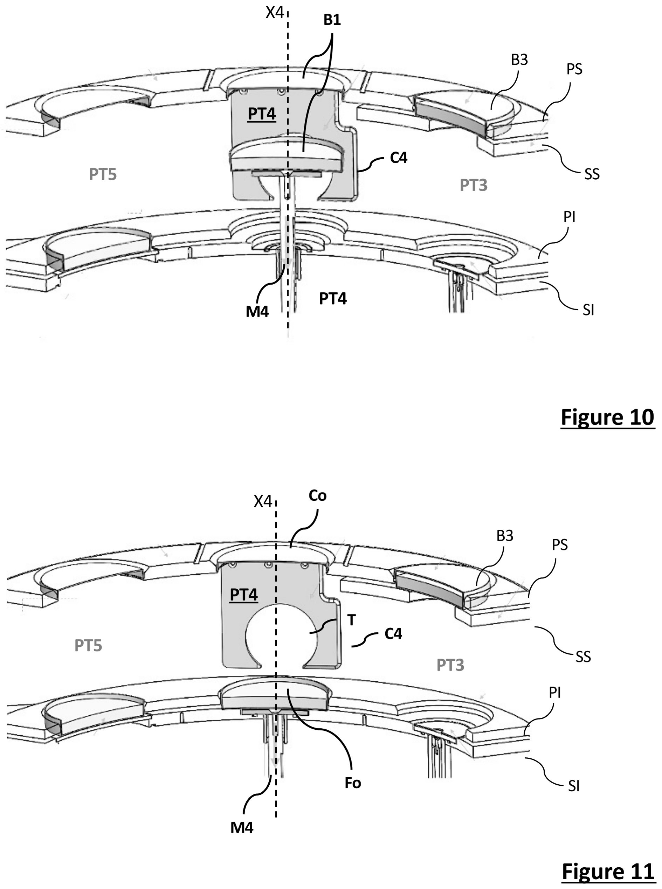

Le poste PT4 comprend un clapet C4 monté mobile entre une position de maintien du fond solidaire du couvercle **(****figure 8****)** et une position de libération permettant une désolidarisation du fond par rapport au couvercle **(****figures 10-11****).** Le poste PT4 comprend en outre un manipulateur M4 monté mobile en translation le long de l'axe X4 entre une position de prélèvement du fond **(****Figure 9****)** et une position de dépose du fond **(****Figure 11****)** sur la platine inférieure PI. Le clapet C4 comprend une lumière d'introduction T du manipulateur M4. La position de prélèvement est prise lorsque le clapet C4 est dans la position de maintien du fond après passage du manipulateur M4 à travers la lumière d'introduction T du clapet C4. Le manipulateur M4 coopère avec un moteur d'entrainement (non représenté), piloté par l'unité de pilotage, qui lorsqu'il est actionné entraîne le déplacement vertical de celui-ci, entre la position de prélèvement et la position de dépose du fond.

Comme illustré à la **figure 8****,** à l'arrivée de la boîte de Petri, le clapet mobile C4 se trouve dans une position horizontale, en continuité de la sole de glissement SS (c'est-à-dire s'étendant sous la surface inférieure de la platine supérieure PS) afin d'assurer le maintien du fond Fo solidaire du couvercle Co (seul le couvercle Co étant en réalité retenue par le logement supérieur Ls1 de la double platine d'entrainement). À ce stade, la fonction de la trappe est d'éviter que le fond Fo de la boîte ne tombe, par effet de gravité, du fait de l'absence de la sole de glissement au niveau du poste PT4. Le manipulateur M4, situé dans l'axe de la lumière d'introduction T du clapet, se trouve dans la position basse située en dessous de la platine inférieure PI, dans le prolongement de la sole de glissement SI.

Puis, comme illustré à la **figure 9****,** le manipulateur M4 est actionné par l'unité de pilotage pour entrainer le déplacement en translation vertical de celui-ci jusqu'à ce qu'il entre en contact avec la boîte de Petri B1. A ce moment, le manipulateur M4 est en position haute et assure le maintien du fond Fo de la boîte de Petri (position de prélèvement). Quant au clapet mobile C4, ce dernier est actionné par l'unité de pilotage pour passer de sa position de maintien (position horizontale) à sa position de libération du fond de la boîte (position verticale) par un mouvement de rotation autour d'un axe horizontal. Une désolidarisation du fond Fo par rapport au couvercle Co de la boîte peut alors être opérée, le fond Fo reposant désormais sur le manipulateur M4.

Enfin, comme illustré aux **figures 10** et **11****,** le manipulateur M4 est à nouveau actionné par l'unité de pilotage pour entrainer son déplacement depuis sa position de prélèvement à sa position de dépose du fond Fo sur la platine inférieure du dispositif d'entrainement. L'ouverture de la boîte de Petri se fait naturellement par effet de gravité car rien ne retient le fond Fo de boîte à son couvercle Co qui reste en appui sur les rebords du logement supérieur Ls1. Cette phase de désolidarisation est schématisée à la **figure 12****.** Une fois la position de dépose prise par le manipulateur M4, le fond Fo repose dans le logement inférieur Li1 de la double platine d'entrainement, alors que le couvercle Co de la boîte est maintenu dans le logement supérieur Ls1 dont l'axe vertical est confondu avec celui du logement inférieur Li1.

A ce stade, l'unité opératoire permet donc de séparer le fond du couvercle (contenant l'échantillon à analyser) de la boîte de Petri, tout en conservant l'appariement de ces deux éléments tout au long du cycle de traitement grâce à la double platine d'entraînement. De fait, même en cas d'arrêt inopiné de la machine, il reste facile de réassembler la boîte de Petri sans risquer d'intervertir les fonds de boite et les couvercles entre eux. Le risque d'erreurs sur les résultats de traitement est donc réduit.

En outre, le poste PT4 est configuré pour évacuer la boîte de Petri lorsque celle-ci est considérée comme problématique par l'unité de traitement. On entend par « problématique », une boîte de Petri qui n'a pas pu être identifiée au poste PT2 et/ou PT3 ou une boîte de Petri dont l'ouverture n'a pas pu se faire au poste PT4. Pour cela, le manipulateur M4 est en outre monté mobile en translation, le long de l'axe X4, entre la position de prélèvement précitée ou encore la position de dépose précitée et une position d'évacuation dans la colonne réceptrice CL1. La position de prélèvement du manipulateur M4 constitue dont une position intermédiaire entre une position basse de dépose et une position haute d'évacuation. Dans le cas d'une boîte de Petri non-identifiée par exemple, le clapet n'est pas activé et reste dans sa position initiale de maintien du fond solidaire du couvercle. Le mécanisme d'évacuation d'une boîte de Petri « problématique » est alors initié (dont le principe est identique à celui décrit plus loin pour le poste PT6).

L'unité de traitement est configurée pour activer le manipulateur M4 et/ou le clapet C4 conformément aux principes décrits ci-dessus, en fonction du résultat d'identification obtenu au préalable pour la boîte de Petri. La double platine est ensuite actionnée par l'unité de pilotage pour entrainer la boîte de Petri (fond Fo et couvercle Co) d'un pas angulaire de 2π/8 autour de l'axe X2 afin d'être acheminée au poste PT5. Le clapet C4 reprend ensuite sa position initiale de maintien.

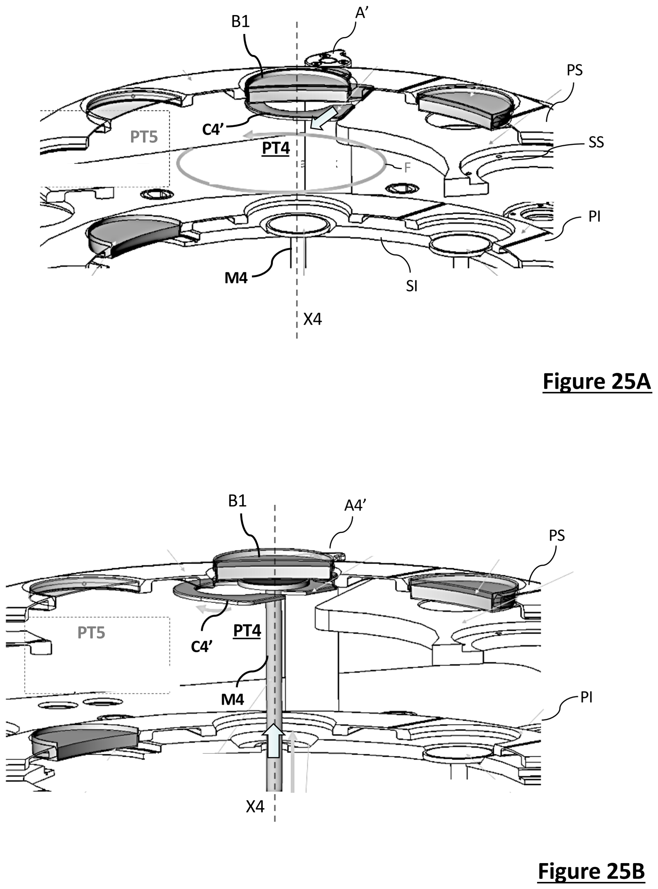

Une variante de mise en œuvre de l'étape 4 est décrite ci-après, en relation avec les **figures 24A, 24B****,** **25A à 25E.** Elle a pour effet de faciliter l'ouverture des boîtes de Petri, en particulier dans les cas où le couvercle se retrouve accroché le fond de la boîte de Petri avec des difficultés de désolidarisation. A la différence de l'exemple de mise en œuvre décrit ci-dessus (en relation avec les figures 8 à 12), le clapet C4' est monté mobile entre deux positions horizontales : une position de maintien du fond solidaire du couvercle **(****Figure 24A****)** et une position de libération permettant une désolidarisation du fond par rapport au couvercle **(****Figure 24B****).** Le clapet C4' comprend une lumière d'introduction du manipulateur M4. La position de prélèvement est prise lorsque le clapet C4' est dans la position de maintien du fond après passage du manipulateur M4 à travers la lumière d'introduction T.

A l'arrivée de la boîte de Petri B1, le clapet mobile C4' se trouve dans une position horizontale, en continuité de la sole de glissement SS s'étendant sous la surface inférieure de la platine supérieure PS) afin d'assurer le maintien du fond solidaire du couvercle (Figure 25A). À ce stade, la fonction du clapet est d'éviter que le fond de la boîte ne tombe, par effet de gravité, du fait de l'absence de la sole de glissement au niveau du poste PT4. Le manipulateur M4, situé dans l'axe de la lumière d'introduction du clapet, se trouve dans la position basse située en dessous de la platine inférieure PI.

Le manipulateur M4 est actionné par l'unité de pilotage pour entrainer le déplacement en translation vertical de celui-ci jusqu'à ce qu'il entre en contact avec la boîte de Petri. A ce moment, le manipulateur M4 est en position haute et assure le maintien du fond de la boîte de Petri (position de prélèvement - Figure 25B). Le clapet C4' est actionné par l'unité de pilotage pour passer de sa position de maintien à sa position de libération du fond de la boîte par un mouvement de rotation au tour d'un axe vertical qui amène le clapet en dehors du dispositif d'entrainement en rotation comme illustré sur la figure 24B. Sur l'axe vertical du clapet est solidarisé un actionneur A4' monté mobile en rotation autour de ce même axe et configuré pour exercer une pression sur un pion (ou un doigt) D4' disposé dans une ouverture traversant le dispositif d'entrainement depuis son bord périphérique jusqu'au logement concerné. Le pion D4' est disposé de manière à être au-dessus de chacun des rebords des logements supérieurs Ls du dispositif d'entrainement comme illustré sur la figure 24B. En position de libération, la pression exercée sur le pion D4' conduit à exercer une pression sur le couvercle de la boite de Petri : le couvercle se trouve alors coincé entre le rebord du logement supérieur Ls et le pion D4', déformant élastiquement le couvercle de manière à créer un flux d'air entrant dans la boite de Petri. Ce flux d'air va contribuer à désolidariser le couvercle du fond de la boîte de Petri en plus de la déformation du couvercle. L'appui du pion sur le couvercle est maintenu une durée prédéfinie contrôlée par l'unité de pilotage à la suite de laquelle le clapet C4' effectue un léger mouvement de rotation vers la droite, sur ordre de l'unité de pilotage. Ce léger mouvement de rotation du clapet supprime la pression du pion D4' sur le couvercle, ce dernier n'étant alors plus coincer dans le logement supérieur du dispositif d'entrainement en rotation. Grâce à cette double pression, une désolidarisation du fond par rapport au couvercle de la boîte peut alors être opérée de manière aisée, le fond reposant désormais librement sur le manipulateur M4. Cette variante de mise en œuvre est particulièrement bien adaptée dans des cas où des difficultés de désolidarisation du couvercles peuvent apparaître.

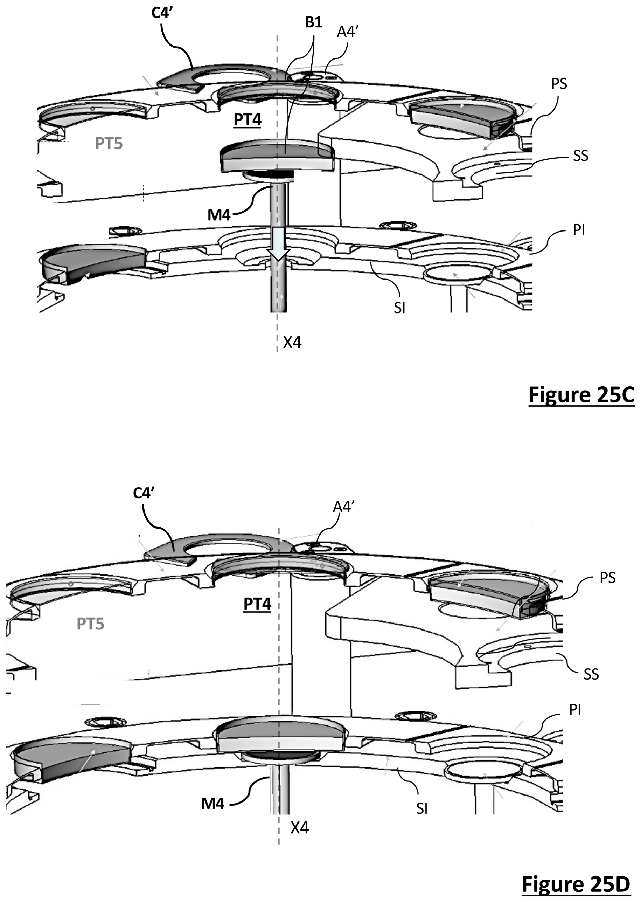

Le manipulateur M4 est à nouveau actionné par l'unité de pilotage pour entrainer son déplacement depuis sa position de prélèvement à sa position de dépose du fond sur la platine inférieure du dispositif d'entrainement (Figure 25C). L'ouverture de la boîte de Petri est naturellement opérée par effet de gravité car rien ne retient le fond de boîte à son couvercle qui reste en appui sur les rebords du logement supérieur Ls1. Une fois la position de dépose prise par le manipulateur M4, le fond repose dans le logement inférieur Li1 de la double platine d'entrainement, alors que le couvercle de la boîte est maintenu dans le logement supérieur Ls1 (Figure 25D).

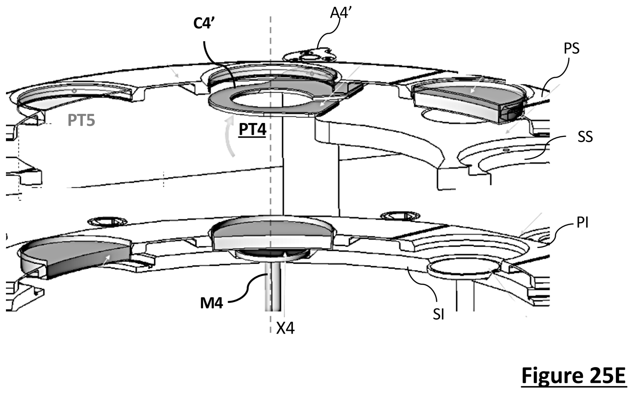

Puis le clapet C4' est à nouveau actionné par l'unité de pilotage pour retourner à sa une position de départ dans le dispositif d'entrainement par un mouvement de rotation au tour de l'axe vertical de l'actionneur A4' (Figure 25E).

### Étape 5 : Analyse du contenu de la boîte de Petri (Figure 13)

La cinquième étape consiste à analyser l'échantillon alimentaire contenu dans la boîte de Petri afin de lui attribuer un type positif ou négatif en fonction de la présence ou non des pathogènes recherchés. Cette cinquième étape est réalisée lorsque la boîte de Petri est positionnée au poste PT5.

Le poste PT5 est équipé d'un module d'imagerie comprenant une source de lumière BL, un capteur d'image CI et un miroir optiquement réfléchissant M'. Ces éléments sont placés dans une enceinte optiquement opaque pour bloquer tout phénomène optique qui viendrait perturber l'analyse. L'unité de traitement est électriquement connectée à la source de lumière BL et au capteur d'image CI à des fins de pilotage de ces éléments et de traitement des résultats. La source de lumière BL, le capteur d'image CI et le miroir réfléchissant M' sont disposés de façon à permettre une analyse par transmission de lumière à travers le fond de boîte Fo. La source de lumière BL est disposée en configuration du type rétro-éclairage (ou *« backlight »* en anglais) par rapport à l'échantillon. Dans le présent exemple, la source de lumière BL est une diode électroluminescente qui émet une lumière blanche d'éclairement compris entre 800 et 6500 Lux. Le capteur d'image CI est configuré pour détecter l'intensité lumineuse reçue en provenance de l'échantillon contenu dans le fond de boîte Fo, et la convertir en signal électrique à destination de l'unité de pilotage. Dans le présent exemple, le capteur d'image CI est un capteur CMOS.

L'unité de traitement est configurée pour piloter, via l'unité de pilotage, le dispositif d'imagerie de façon à permettre l'acquisition d'une image ou d'une série d'images de l'échantillon, l'analyse des images acquises et l'attribution du type de boîte donné en fonction des résultats d'analyse.

La phase d'analyse consiste en la recherche, par analyse d'image, de la présence de la bactérie listeria monocytogenes au sein de l'échantillon. Si le résultat d'analyse est positif à la présence du microorganisme recherché, la boîte de Petri B1 se voit attribuer le type « positif ». Si le résultat d'analyse est négatif, la boîte de Petri B1 se voit attribuer le type « négatif ». Si le résultat d'analyse est indéterminé, la boîte de Petri B1 se voit attribuer le type « positive » également.

Par ailleurs, on rappelle que si la boîte de Petri B1 n'a pas pu être identifiée aux postes de lecture PT2 ou PT3, la boîte de Petri B1 se voit attribuer le type « problématique ». Et si la boîte de Petri B1 n'a pas pu être ouverte aux postes d'ouverture PT4, elle se voit également attribuer le type « problématique ». La boîte de Petri est triée par la machine en fonction du type de boîte qui lui aura été attribuée par l'unité de traitement.

Selon une mise en œuvre particulière, la phase d'analyse comprend en outre la détection de la présence de colonies de microorganismes et la détermination du nombre de colonies présentes dans l'échantillon. Ainsi, à un échantillon donné identifié par son code-barres, lui est attribué le type de boîte ainsi qu'une information sur le nombre de colonies détectées. Ces données sont stockées dans la mémoire de l'unité de traitement.

Bien entendu, diverses méthodes connues de l'Homme du Métier de détection ou de reconnaissance d'objets dans une image peuvent être utilisées pour procéder à l'analyse d'image et la recherche de colonies de micro-organismes, telles qu'une méthode d'apprentissage basée sur un processus d'intelligence artificielle (par exemple : apprentissage automatique (« *Deep Learning* »), apprentissage automatique profond (ou *« Machine Learning* »), apprentissage supervisé) ou une méthode basée sur l'application d'un filtre numérique prédéfini, la segmentation d'image, la reconnaissance de texture, ou encore sur une méthode d'autocorrélation de caractéristiques d'image. L'Homme du Métier, connaissant ces méthodes, est en mesure d'adapter l'algorithme exécuté à cette phase du procédé, en particulier en fonction de la nature de l'analyse à réaliser, du taux de faux positifs ou de faux négatifs souhaités.

Le module d'imagerie peut bien entendu être configuré d'autres manières et comprendre des sources de lumière additionnelles afin d'optimiser l'éclairage et les effets lumineux pour faciliter la recherche des microorganismes.

La double platine est ensuite actionnée par l'unité de pilotage pour entrainer la boîte de Petri (fond Fo et couvercle Co séparés) d'un pas angulaire de 2π/8 autour de l'axe X2 afin d'être acheminée au poste PT6.

### Étape 6 : Évacuation des boîtes du type « positif » (Figures 14 à 18)

Cette sixième étape consiste à réassembler la boîte de Petri et évacuer celle-ci vers le carrousel de stockage CS2, en cas de résultat positif à la présence de listeria monocytogenes. Cette sixième étape est réalisée lorsque la boîte de Petri est positionnée au poste PT6 et lorsque celle-ci est du type « positif ».

Le poste PT6 comprend un manipulateur M6 monté mobile en translation le long de l'axe d'évacuation X6 entre une position de prélèvement du fond Fo depuis la platine inférieure PI **(****Figures 14-15****),** une position intermédiaire de solidarisation du fond Fo avec le couvercle Co de la boîte au niveau de la platine supérieure PS **(****Figure 16****)** et une position de dépose de la boîte solidarisée dans le carrousel de stockage CS2 **(****Figures 17-18****).** Le manipulateur M6 coopère avec un moteur d'entrainement (non représenté), piloté par l'unité de pilotage, qui lorsqu'il est actionné entraîne le déplacement vertical de celui-ci, depuis une position basse vers une position haute et inversement depuis la position haute vers la position basse. Le manipulateur M6 est dimensionné pour être logé dans la sole de glissement inférieure SI et dont la course passe au travers des ouvertures des platines inférieure PI et supérieure PS.

A l'arrivée au poste PT6, le fond de boîte Fo est disposé sur la platine inférieure PI tandis que le couvercle Co est disposé sur la platine supérieure PS **(****figures 14** et **15****).** Le manipulateur M6 est alors encastré dans la sole de glissement inférieure SI pour ne pas gêner le déplacement du fond Fo sur la sole de glissement SI. Sur commande de l'unité de pilotage, le moteur d'entrainement du manipulateur M6 est actionné pour entrainer le déplacement vertical de celui-ci et la montée du fond Fo en direction de la colonne de stockage C6 du carrousel CS2. Au fur-et-à-mesure de la montée, le manipulateur M6 passe au travers de l'ouverture de la platine supérieure PS de sorte que le fond Fo entre en contact avec le couvercle Co resté sur la platine supérieure PS et se solidarise à celui-ci relativement sans frottement. Afin d'assurer à un réassemblage fond-couvercle en douceur, la vitesse du manipulateur est ralentie au passage de la platine supérieure PS **(****figure 16****).** Le manipulateur M6 continue sa montée pour passer au travers de l'ouverture de la plaque inférieure du carrousel CS2, amenant la boîte de tri réassemblée à passer à travers, puis au-dessus, d'une paire de crochets anti-retours Cr1-Cr2 pour être empilée avec les autres boîtes de Petri positives préalablement traitées. Ces crochets anti-retours sont montés sur charnière pour permettre la réalisation d'une liaison pivot passive par rapport à la plaque du carrousel, les rendant mobiles entre une position horizontale assurant le maintien de la boîte dans la colonne de stockage CS6 **(****figure 18****)** et une position oblique (voire quasi-verticale) autorisant l'évacuation de la boîte dans la colonne de stockage CS6 **(****Figure 16**).

Si la boîte de Petri est d'un autre type, celle-ci n'est pas évacuée par le poste PT6. Le manipulateur M6 n'est pas activé, et le fond de boîte Fo et le couvercle Co associé restent dans leur logement respectif.

La double platine est ensuite actionnée par l'unité de pilotage pour entrainer la boîte de Petri (fond Fo et couvercle Co séparés) d'un pas angulaire de 2π/8 autour de l'axe X2 afin d'être acheminée au poste PT7.

### Étape 7 : Évacuation des boîtes du type « positif » (Figure 14)

Cette septième étape est réalisée lorsque la boîte de Petri est positionnée au poste PT7. Cette septième étape est identique à la sixième étape et est mise en œuvre pour une boîte de Petri « positive » et en coopération avec le second carrousel de stockage CS2, lorsque la colonne de stockage C6 est pleine (soit quarante boîtes empilées ici) et ne peut donc pas accueillir d'autres boîtes de Petri. Dans ce mode de réalisation particulier, le poste PT7 a donc la même fonction que le poste PT6 et intervient en doublon lorsque la colonne de stockage associé au poste PT6 est pleine, ce qui permet d'augmenter la capacité de stockage de boîtes positives (soit quatre-vingts boîtes ici). L'unité de traitement est configurée pour gérer le comptage des boîtes de Petri positives évacuées par le poste PT6 dans la colonne CL2 avant d'activer le poste PT7 pour l'évacuation d'autres boites de Petri positives dans la colonne CL3.

Le poste PT7 comprend un manipulateur M7 identique au manipulateur M6, c'est-à-dire monté mobile en translation le long de l'axe d'évacuation X7, coopérant avec un moteur d'entrainement (non représenté), piloté par l'unité de pilotage. Le manipulateur M7 est dimensionné pour être logé dans la sole de glissement inférieure SI et dont la course passe au travers des ouvertures des platines inférieure PI et supérieure PS. Sur commande de l'unité de pilotage, l'actionnement du moteur d'entrainement entraine le déplacement vertical du manipulateur M7 et la montée du fond Fo en direction de la colonne de stockage C6 du carrousel CS2, passant au travers de l'ouverture de la platine supérieure PS pour se solidariser à son couvercle, puis passant au travers de l'ouverture de la plaque inférieure du carrousel CS2, pour amener la boîte de tri réassemblée dans la colonne de stockage C7 au-dessus d'une paire de crochets anti-retours.

Si la boîte de Petri est d'un autre type, celle-ci n'est pas évacuée par le poste PT7. Le manipulateur M7 n'est pas activé, et le fond de boîte Fo et le couvercle Co associé restent dans leur logement respectif.

La double platine est ensuite actionnée par l'unité de pilotage pour entrainer la boîte de Petri (fond Fo et couvercle Co séparés) d'un pas angulaire de 2π/8 autour de l'axe X2 afin d'être acheminée au poste PT8.

### Étape 8 : Évacuation des boîtes du type « négatif » (Figure 23)

Cette huitième étape est réalisée lorsque la boîte de Petri est positionnée au poste PT8. Cette huitième étape est identique à la sixième (ou septième) étape et est mise en œuvre pour une boîte de Petri « négative » en coopération avec le premier carrousel de stockage CS1.

Le poste PT8 comprend un manipulateur M8 identique au manipulateur M6 (ou M7), c'est-à-dire monté mobile en translation le long de l'axe d'évacuation XR, coopérant avec un moteur d'entrainement (non représenté), actionné par l'unité de pilotage lorsqu'une boîte de Petri « négative » est présente à ce poste. Le manipulateur M8 est dimensionné pour être logé dans la sole de glissement inférieure SI et dont la course passe au travers des ouvertures des platines inférieure PI et supérieure PS. Sur commande de l'unité de pilotage, l'actionnement du moteur d'entrainement entraine le déplacement vertical du manipulateur M8 et la montée du fond Fo en direction de la colonne réceptrice du carrousel CS1, passant au travers de l'ouverture de la platine supérieure PS pour se solidariser à son couvercle, puis passant au travers de l'ouverture OR de la plaque inférieure du carrousel CS1 pour amener la boîte de tri réassemblée dans la colonne de stockage et y être empilée avec les autres boîtes de Petri négatives préalablement traitées.

La double platine est ensuite actionnée par l'unité de pilotage pour entrainer les boîtes de Petri d'un pas angulaire de 2π/8 autour de l'axe X2 tant que des boîtes de Petri restent à traiter.

Ainsi, la machine MT est conçue pour que, en fonction du type attribué à la boîte de Petri, celle-ci est évacuée vers le premier ou le second carrousel de stockage, ce qui permet de réaliser un tri automatique des boîtes de Petri.

En pratique, il est possible de réaliser un traitement complet sur un ensemble de quatre cents boîtes, de manière autonome une fois que les boites chargées dans le carrousel de stockage CS1, et ce grâce à cette coopération astucieuse entre le carrousel de stockage CS1 mobile et le dispositif d'entrainement DER, activés de manière synchrone pour chaque pile de boîtes de Petri à distribuer. Le nombre de boîtes est simplement donné ici à titre illustratif et un nombre plus ou moins important peut être bien entendu envisagé, sans sortir du cadre de l'invention, en fonction de la capacité de stockage du carrousel CS1. Cette capacité de stockage dépend de la hauteur et du nombre de colonnes de stockage.

On notera par ailleurs que la machine MT compte autant de boîtes de Petri en cours de traitement que de postes de travail compris dans l'unité opératoire, tous les postes de travail fonctionnant en permanence sauf lorsque le dispositif d'entrainement est actionné pour entrainer en rotation les boîtes de Petri par rapport aux différents postes de travail.

On présente maintenant, en relation avec la **figure 21****,** une variante de réalisation du dispositif d'entrainement de boîtes de Petri selon l'invention. A la différence du dispositif d'entrainement DER conformé pour accueillir des boîtes de Petri à l'endroit **(****figure 3****),** ce dispositif d'entrainement DER-2 est conformé pour accueillir des boîtes de Petri disposées à l'envers, c'est-à-dire le couvercle orienté vers le bas et le fond orienté vers le haut. Cette variante de réalisation est particulièrement intéressante par exemple lorsque l'analyse requiert que les boîtes de Petri restent fermées tout au long du process afin d'éviter tout risque de contamination du milieu de culture. Par exemple, pour l'analyse de moisissures, il est essentiel que les milieux de culture ne soient pas contaminés par des spores hautement volatiles pouvant être présents en surface des boîtes de Petri. Par ailleurs, les filaments de moisissures recouvrant toute ou partie de la surface de la boite de Petri et pouvant recouvrir différentes colonies de moisissures, il est très difficile d'identifier et de différencier des moisissures de types différents. La lecture par le fond de la boîte de Petri, en la retournant, permet de mieux différencier la présence de plusieurs types de moisissures car leurs colonies apparaissent plus clairement situées sous les filaments par une observation par le dessous de la boîte de Petri.

Comme pour le dispositif DER, le dispositif DER-2 comprend une double platine d'entrainement composée d'une platine d'entrainement supérieure PS-2 et d'une platine d'entrainement inférieure PI-2, de forme globalement annulaire, disposées en parallèle et solidaires l'une de l'autre par des entretoises. Le dispositif DER-2 comprend également une sole de glissement supérieure SS-2 s'étendant partiellement sous la platine PS-2 et une sole de glissement inférieure SI-2 s'étendant sous la platine PI-2. Les soles de glissement SS-2 et SI-2 sont identiques à celles du dispositif DER-2. Les platines d'entrainement PS-2 et PI-2 sont dotées chacune d'une série de huit logements identiques régulièrement répartis en périphérie du dispositif et alignés verticalement deux-à-deux.

A la différence du dispositif DER, la série des huit logements supérieurs du dispositif DER-2 destinés à accueillir les boîtes de Petri ne possèdent pas de rebord de retenue de couvercle (i.e. d'épaulement annulaire EA). En effet, les huit logements de la platine PS-2 comprennent chacun un trou traversant ayant une première portion de forme conique (conduisant à une réduction de section progressive) et une deuxième portion cylindrique dimensionnée pour permettre le passage de la boîte de Petri dans son ensemble (fond et couvercle) à travers le logement. Ainsi, pour une demande de traitement de boîtes de Petri à l'envers, il suffit de retirer la double platine d'entrainement PS-2 - PI-2 du dispositif DER-2 pour la remplacer avec la double platine d'entrainement PS - PI du dispositif DER, les soles de glissement restant identiques d'un mode de réalisation à l'autre.

La **figure 22** représente, de manière schématique et simplifiée, la structure d'un dispositif de traitement de boîtes de Petri dans un mode de réalisation particulier, par exemple l'unité de traitement mettant en œuvre le procédé selon l'invention (par exemple les étapes 1 à 8 décrites ci-dessus en relation avec les **figures 5** à **20****, 23).**

Ce dispositif de traitement 10 comprend une mémoire vive 130 (par exemple une mémoire RAM), une unité CPU 110, équipée par exemple d'un processeur ou microprocesseur, et pilotée par un programme d'ordinateur stocké dans une mémoire morte 120 (par exemple une mémoire ROM ou un disque dur). À l'initialisation, les instructions de code du programme d'ordinateur sont par exemple chargées dans la mémoire vive 130 avant d'être exécutées par le processeur de l'unité CPU 110. Un tel programme d'ordinateur permet l'exécution d'au moins une itération des étapes 1 à 8 décrites ci-dessus (c'est-à-dire au moins un cycle de traitement).

L'unité de traitement 51 reçoit en entrée des consignes ainsi que des instructions de démarrage du traitement (représentées par la flèche E) renseignées par l'opérateur via l'interface logicielle. Le processeur de l'unité CPI 51 exécute alors le procédé à partir des consignes d'entrée E et délivre en sortie les commandes de pilotage correspondantes (représentées par la flèche S) à destination des différents postes de traitement, selon les instructions du programme 120, à l'aide de l'unité de pilotage de la machine.

Cette **figure 22** illustre seulement une manière particulière, parmi plusieurs possibles, de réaliser les différents étapes détaillées ci-dessus. En effet, le procédé de traitement selon l'invention se réalise indifféremment :
- sur une machine de calcul reprogrammable (un ordinateur PC, un processeur DSP ou un microcontrôleur) exécutant un programme comprenant une séquence d'instructions ; ou
- sur une machine de calcul dédiée (par exemple un ensemble de portes logiques comme un FPGA ou un ASIC, ou tout autre module matériel).

Dans le cas où l'invention est implantée sur une machine de calcul reprogrammable, le programme correspondant (c'est-à-dire la séquence d'instructions) pourra être stocké dans un médium de stockage amovible (tel que par exemple une disquette, un CD-ROM ou un DVD-ROM) ou non, ce médium de stockage étant lisible partiellement ou totalement par un ordinateur ou un processeur.

## Revendications

1. Machine automatisée de traitement de boîtes de culture, **caractérisée en ce qu'**elle comprend :
- un premier carrousel de stockage (CS1) monté mobile en rotation autour d'un premier axe (X1) et destiné au stockage de boîtes de culture à traiter;
- un second carrousel de stockage (CS2) monté fixe autour d'un second axe (X2) ;
- une unité opératoire destinée à recevoir les boîtes de culture en succession continue depuis le premier carrousel et à effectuer une série de traitements successifs sur chacune des boîtes de culture reçues, ladite unité opératoire comprenant :
∘ une pluralité de postes de traitement stationnaires (PT1-PT8) répartis selon un pas prédéterminé autour du second axe, au moins une partie desdits postes de traitement étant susceptibles d'être activés de façon au moins partiellement concomitante, lesdits postes de traitement comprenant :
▪ un poste de réception destiné à recevoir une boîte de culture à traiter depuis le premier carrousel ;
▪ un poste d'analyse et d'attribution destiné à analyser le contenu de la boîte de culture reçue et à lui attribuer un type donné parmi au moins deux types de boîte prédéfinis en fonction des résultats d'analyse ;
· au moins un premier et un deuxième poste d'évacuation destinés à évacuer la boîte de culture vers le premier ou le second carrousel de stockage en fonction du type attribué à ladite boîte de culture ;
o un dispositif d'entrainement en rotation (DER) des boîtes de culture par rapport aux postes de traitement, ledit dispositif d'entrainement comprenant des logements de boîtes de culture répartis selon un pas correspondant au pas des postes de traitement de sorte qu'une rotation du dispositif d'entrainement autour du second axe amène une boîte de culture donnée successivement sur chacun des postes de traitement.

2. Machine selon la revendication 1, dans laquelle ledit dispositif d'entrainement comprend une platine d'entrainement supérieure (PS) et une platine d'entrainement inférieure (PI) disposées en parallèle l'une de l'autre et solidaires l'une de l'autre, les platines d'entrainement supérieure et inférieure étant montées mobiles en rotation autour du second axe et comprenant un ensemble de logements supérieurs (Ls1-Ls8) et inférieurs (Li1-Li8) de boîtes de Petri respectivement, les logements supérieurs et inférieurs étant régulièrement répartis en périphérie des platines d'entrainement supérieure et inférieure autour du second axe, et disposés en regard deux à deux.

3. Machine selon la revendication 2, dans laquelle ledit dispositif d'entrainement comprend en outre :
- une sole de glissement supérieure (SS) montée fixe par rapport au second axe et s'étendant partiellement en face inférieure de la platine d'entrainement supérieure, et
- une sole de glissement inférieure (SI) montée fixe par rapport au second axe et s'étendant en face inférieure de la platine d'entrainement inférieure.

4. Machine selon la revendication 3, où les boîtes de culture à traiter sont munies chacune d'un couvercle solidarisé à un fond et disposées dans une position dite à l'endroit dans le premier carrousel et dans laquelle :
- les logements supérieurs sont dotés chacun d'un trou traversant dimensionné pour le passage d'une boîte de culture et dont l'extrémité inférieure se termine par un épaulement annulaire dimensionné pour retenir le couvercle dans le logement supérieur et autoriser une désolidarisation du fond par rapport au couvercle par effet de gravité en l'absence de la sole de glissement supérieure, la sole de glissement supérieure s'étendant partiellement sous la platine d'entrainement supérieure pour maintenir le fond solidaire du couvercle sur au moins un des postes de traitement ;
- les logements inférieurs sont dotés chacun d'un trou traversant dimensionné pour le passage d'un fond de boîte de culture, la sole de glissement inférieure s'étendant sous la platine d'entrainement inférieure pour maintenir le fond dans le logement inférieur ;

5. Machine selon la revendication 4, dans laquelle les postes de traitement stationnaires comprennent en outre un poste d'ouverture de boîte de culture (PT4) comprenant :
- un clapet (C4) monté mobile entre au moins une position de maintien du fond solidaire du couvercle et une position de libération permettant une désolidarisation du fond par rapport au couvercle ;
- un manipulateur (M4) monté mobile en translation entre au moins une position de prélèvement du fond et une position de dépose du fond sur la platine inférieure ;
le clapet comprenant une lumière d'introduction du manipulateur, ladite position de prélèvement étant prise lorsque le clapet est dans la position de maintien du fond après passage du manipulateur à travers la lumière d'introduction du clapet.

6. Machine selon la revendication 5, dans laquelle le clapet monté mobile en rotation autour d'un axe vertical d'un actionneur lequel est configuré pour exercer une pression sur un pion disposé dans une ouverture traversant le dispositif d'entrainement, ledit pion exerçant une pression sur le couvercle lorsque le clapet est en position de libération.

7. Machine selon l'une quelconque des revendications 3 à 6, dans laquelle lesdits au moins un premier et un deuxième postes d'évacuation comprennent chacun un organe manipulateur (M6, M7) monté mobile en translation entre au moins une position de prélèvement du fond depuis la platine d'entrainement inférieure, une position intermédiaire de solidarisation du fond avec le couvercle au niveau de la platine d'entrainement supérieure, et une position de dépose du fond et du couvercle solidarisés dans le premier ou le second carrousel de stockage.

8. Machine selon l'une quelconque des revendications 3 à 7, dans laquelle les postes de traitement stationnaires comprennent en outre au moins un poste d'identification de boîte de culture appartenant au groupe comprenant : un poste d'identification à lecteur de code-barre frontal (PT2) et un poste d'identification à lecteur de code-barre latéral (PT3), ladite sole de glissement supérieure (SS) couvrant la face inférieure de la platine d'entrainement supérieure sur le poste de réception et ledit au moins un poste d'identification.

9. Machine selon l'une quelconque des revendications 1 à 8, ledit au moins un premier poste d'évacuation est destiné à évacuer vers le premier carrousel les boîtes de culture pour lesquelles un premier type est attribué et ledit au moins un deuxième poste d'évacuation est destiné à évacuer vers le second carrousel les boîtes de culture pour lesquelles est attribué un deuxième type.

10. Machine selon la revendication 9, dans laquelle le poste d'ouverture de boîte de culture est en outre destiné à évacuer vers le premier carrousel les boîtes de culture pour lesquelles un troisième type est attribué relatif à une problématique de traitement, le manipulateur (M4) dudit poste d'ouverture étant en outre monté mobile en translation entre une position, parmi ladite position de prélèvement et ladite position de dépose, et une position d'évacuation de boîte dans le second carrousel de stockage.

11. Machine selon la revendication 3, où les boîtes de culture à traiter sont munies chacune d'un couvercle solidarisé à un fond et disposées dans une position dite à l'envers dans le premier carrousel, et dans laquelle :
- les logements supérieurs sont dotés chacun d'un trou traversant dimensionné pour le passage d'une boîte de culture, la sole de glissement supérieure s'étendant partiellement sous la platine d'entrainement supérieure pour maintenir la boîte de culture fermée en position à l'envers dans le logement supérieur sur au moins un des postes de traitement ;
- les logements inférieurs sont dotés chacun d'un trou traversant dimensionné pour le passage d'une boîte de culture, la sole de glissement inférieure s'étendant sous la platine d'entrainement inférieure pour supporter le fond maintenir la boîte de culture fermée en position à l'envers dans le logement inférieur;

12. Procédé de traitement de boîtes de culture au moyen d'une machine de traitement automatisée comprenant un premier carrousel de stockage (CS1), un second carrousel de stockage (CS2), et une unité opératoire destinée à recevoir les boîtes de culture en succession continue et à effectuer une série de traitements successifs sur chacune des boîtes de culture, l'unité opératoire comprenant une pluralité de postes de traitement stationnaires (PT1-PT8) répartis à un pas constant autour d'un axe de rotation et un dispositif d'entrainement en rotation (DER) des boîtes de culture par rapport aux postes de traitement, ledit dispositif d'entrainement comprenant des logements de boîtes de culture répartis selon un pas correspondant au pas des postes de traitement de sorte qu'une rotation du dispositif d'entrainement autour du second axe amène une boîte de culture donnée successivement sur chacun des postes de traitement pour effectuer les étapes suivantes, de façon au moins partiellement concomitante :
- réception d'une boîte de culture à traiter depuis le premier carrousel ;
- analyse du contenu de la boîte de culture reçue et attribution à ladite boîte un type donné parmi au moins deux types de boîte prédéfinis en fonction des résultats d'analyse ;
- évacuation de la boîte de culture dont le contenu a été analysé vers le premier ou le second carrousel de stockage en fonction du type attribué à ladite boîte de culture.

13. Produit programme d'ordinateur, comprenant des instructions de code de programme pour la mise en œuvre du procédé selon la revendication 12, lorsque ledit programme est exécuté sur un ordinateur.

14. Médium de stockage lisible par ordinateur et non transitoire, stockant un produit programme d'ordinateur selon la revendication 13.
